(19) Europäisches Patentamt
European Patent Office
Office européen des brevets

(11) **EP 2 782 056 A1**

(12) **EUROPEAN PATENT APPLICATION**

(43) Date of publication:
**24.09.2014 Bulletin 2014/39**

(21) Application number: **13159813.8**

(22) Date of filing: **18.03.2013**

(51) Int Cl.:
**G06Q 10/06** (2012.01)     **G06Q 50/22** (2012.01)
**G06F 19/00** (2011.01)     **G06F 3/0484** (2013.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Optimal Medicine Ltd
London EC3V 3ND (GB)**

(72) Inventors:
• **Munro, Janet
  34380 St Martin du Londres (FR)**
• **Tubman, Kenneth L
  Millville, MA 01529 (US)**
• **Turner, Rick
  Manchester M1 6DE (GB)**

(74) Representative: **Miller Sturt Kenyon
  9 John Street
  London WC1N 2ES (GB)**

(54) **Personalised medicine system for rating patient characteristics**

(57)     A personalised medicine system comprising: a processor; memory; an input module; and an output module, said personalised medicine system being configured: to provide a plurality of predetermined patient assessment routines; to display a plurality of pages associated with respective stages in a workflow for guiding a clinical consultation; in response to an input from a user on a displayed page, to receive data for a selected patient assessment routine; to determine a value for at least one patient characteristic based on the received data; and to display a respective graphical scale for each of a plurality of patient characteristics, wherein an indicator is displayed on each graphical scale at a position corresponding to at least one of the determined characteristic value and a characteristic value directly input by the user. A method and a computer program are also provided.

Fig. 12

EP 2 782 056 A1

## Description

## Field of the Invention

The present invention relates to a personalised medicine system, and in particular a system that allows a clinician to rate characteristics of a patient.

## Background of the Invention

Rating scales are a well-recognised and useful tool in assessing patient characteristics such as symptoms and side effects. However, due to their often complicated structure it can be difficult to extract meaning from their completion. Moreover, the plethora of overlapping scales, each with its own intrinsic value, can further obfuscate a clinician's overall appreciation of the patient's current and evolving clinical status. In addition, the completion of such rating scales can often be very time consuming, so it is difficult for clinicians to complete them on a regular basis, and clinicians and patients often see such rating scales as an unwelcome imposition. Failure to complete rating scales regularly means that clinicians fail to capture information about the patient's status across multiple clinical parameters, and do not have a method of recording a patient's symptom or side effect severity in relation to previous consultations.

## Summary of the Invention

The present invention seeks to address these shortcomings by providing a medical system that displays a characteristic severity rating value on a graphical scale. Preferably, the system calculates the rating value for a predetermined characteristic and displays the calculated value, as well as allowing a user to input a value manually, taking into account the severity of the previous ratings.

According to a first aspect of the present invention, there is provided a personalised medicine system comprising: a processor; memory; an input module; and an output module, said personalised medicine system being configured: to provide a plurality of predetermined patient assessment routines; to display a plurality of pages associated with respective stages in a workflow for guiding a clinical consultation; in response to an input from a user on a displayed page, to receive data for a selected patient assessment routine; to determine a value for at least one patient characteristic based on the received data; and to display a respective graphical scale for each of a plurality of patient characteristics, wherein an indicator is displayed on each graphical scale at a position corresponding to at least one of the determined characteristic value and a characteristic value directly input by the user.

Preferably, the system is configured to display on each graphical scale both a first indicator at a position corresponding on the determined characteristic value, if available, and a second indicator at a position corresponding to the directly input characteristic value, if available.

Preferably, the system is further configured: to store at least one previously set characteristic value for each graphical scale; and additionally to display a third indicator for the previously set patient characteristic value on each graphical scale.

Preferably, at least one said patient assessment routine comprises a predetermined rating scale, the received data for the patient assessment routine comprises a number between predetermined limits in response to each question in the rating scale that is answered, and the determined value for a characteristic corresponds to the sum of the numbers input in response to questions relevant to the respective characteristic.

In this case, the patient assessment routine may include questions relevant to more than one characteristic.

In addition, more than one patient assessment routine may include a question that is relevant to a respective characteristic. In this case, it is preferred that for each respective characteristic for which there is more than one patient assessment routine that includes a relevant question, the patient assessment routines are ranked in order of importance, and if at least two patient assessment routines that include relevant questions have been completed, the data input in respect of the assessment routine ranked as most important for the characteristic are used to calculate the determined characteristic value.

It is further preferred that the patient assessment routines are ranked in order of the largest possible sum of numbers that can be input in response to questions relevant to the respective characteristic.

Preferably, the indicator is displayed at a position corresponding to a normalised value of the determined characteristic value expressed as one of a ratio and a percentage of the maximum characteristic value that can be determined during the patient assessment routine.

Preferably, the system is configured such that the user drags the indicator along the graphical scale to directly input the characteristic value.

In this case it is preferred that the system is configured to display the indicator at a position based on the determined characteristic value before the user directly inputs the characteristic value.

Preferably, the characteristics include at least one of symptoms of an illness experienced by a patient and side effects of medications experienced by a patient.

Preferably, the system is further configured, in response to a user input, to display the completed assessment routine used to position the indicator.

Preferably, the system is further configured, in response to a user input, to display when an assessment routine used to position the indicator is uncompleted.

Preferably, at least one patient assessment routine comprises input of physical examination data, and the value determined for the at least one patient characteristic is based on a difference between previously input physical examination data and currently input physical examination data.

According to another aspect of the present invention, there is provided a method comprising: providing a plurality of predetermined patient assessment routines; displaying a plurality of pages associated with respective stages in a workflow for guiding a clinical consultation; in response to an input from a user on a displayed page, receiving data for a selected patient assessment routine; determining a value for at least one patient characteristic based on the received data; and displaying a respective graphical scale for each of a plurality of patient characteristics, wherein an indicator is displayed on each graphical scale at a position corresponding to at least one of the determined characteristic value and a characteristic value directly input by the user.

According to a further aspect of the present invention, there is provided a computer program stored on a computer-readable medium for causing a computer to carry out a method comprising: providing a plurality of predetermined patient assessment routines; displaying a plurality of pages associated with respective stages in a workflow for guiding a clinical consultation; in response to an input from a user on a displayed page, receiving data for a selected patient assessment routine; determining a value for at least one patient characteristic based on the received data; and displaying a respective graphical scale for each of a plurality of patient characteristics, wherein an indicator is displayed on each graphical scale at a position corresponding to at least one of the determined characteristic value and a characteristic value directly input by the user.

**Brief Description of the Drawings**

Embodiments of the present invention will now be described by way of further example only and with reference to the accompanying drawings, in which:

Fig. 1 is a schematic diagram of the architecture of a device for use in the present invention;
Fig. 2 is a schematic diagram of a system according to the present invention;
Fig. 3 is a table representing a workflow for use in the present invention;
Fig. 4 is a partial representation of a screen for display in the present invention, including a header, another display page and a guidelines area;
Fig. 5 is a partial representation of an actions from last visit display page for use in the present invention;
Fig. 6 is a partial representation of an actions due display page for use in the present invention;
Fig. 7 is a partial representation of another display screen for use in the present invention, including two display pages and a guidelines area;

Fig. 8 is a partial representation of another display screen for use in the present invention, including a display page and a guidelines area;
Fig. 9 is a partial representation of another display screen for use in the present invention, including a header, a display page and a guidelines area;
Fig. 10 is a partial representation of another display screen for use in the present invention, including a header, a display page and a guidelines area;
Fig. 11 is a representation of a pop-up display for a symptom rating scale for use in the present invention;
Fig. 12 is a partial representation of a symptom severity sliders display page for use in the present invention;
Fig. 13 is a partial representation of side effect rating scale display page for use in the present invention;
Fig. 14 is a partial representation of another display screen for use in the present invention, including a header, a display page and a guidelines area;
Fig. 15 is a partial representation of a side effect severity sliders display page for use in the present invention;
Fig. 16 is a partial representation of another display screen for use in the present invention, including a header, a display page and a guidelines area;
Fig. 17 is a partial representation of another display screen for use in the present invention, including a header, a display page and a guidelines area;
Fig. 18 is a partial representation of a side effect relative risk display page for use in the present invention;
Fig. 19 is a partial representation of a drug information display page for use in the present invention;
Fig. 20A is a partial representation of a timeline for use in the present invention;
Fig. 20B is a partial representation of a visit selection display for use in the present invention;
Fig. 20C is a partial representation of another timeline for use in the present invention;
Fig. 21 is a partial representation of a display screen with a minimised timeline for use in the present invention;
Fig. 22 is a schematic diagram of an advisory information system for use in the present invention;
Fig. 23 is a schematic representation of hierarchical rule for use in the present invention;
Fig. 24 is an exemplary extract of a monitoring rules table for use in the present invention;
Fig. 25 is a partial representation of a monitoring schedule display screen for use in the present invention;
Fig. 26 is an exemplary extract of a table of influences for use in the present invention;
Fig. 27 is an exemplary extract of an individual side effect table for use in the present invention;
Fig. 28 is an exemplary extract of a drug side effects weighting table for use in the present invention;

Fig. 29 is an exemplary extract of a symptoms slider value determination table for use in the present invention;

Fig. 30 is an exemplary extract of a side effects slider value determination table for use in the present invention; and

Fig. 31 is a flow chart illustrating a rule for use in the present invention.

## Detailed Description

### General Architecture

The present invention provides a personalised medicine system, which may be a specialised electronic device having only the function of the personalised medicine system. More usually the personalised medicine system of the present invention is an existing electronic device, such as a computer, adapted to have the functionality required by the present invention. Moreover, the personalised medicine system of the present invention may be distributed between several electronic devices/computers which are connected by one or more of a wired LAN, a wireless LAN, a WAN and the Internet.

Where the personalised medicine system is embodied as single electronic device it may be an existing mobile communications device such as a smart phone (for example iPhone™ or Android™ mobile/cell phone) or a tablet computer (for example iPad™ or Samsung Galaxy™ tablet). Such a mobile communications device can be adapted to have the functionality of a reminder device according to the present invention by downloading an application or widget to the mobile communications device. In some embodiments, it may interact with other devices to provide the functionality. In others, the mobile communications device may operate in a stand alone fashion.

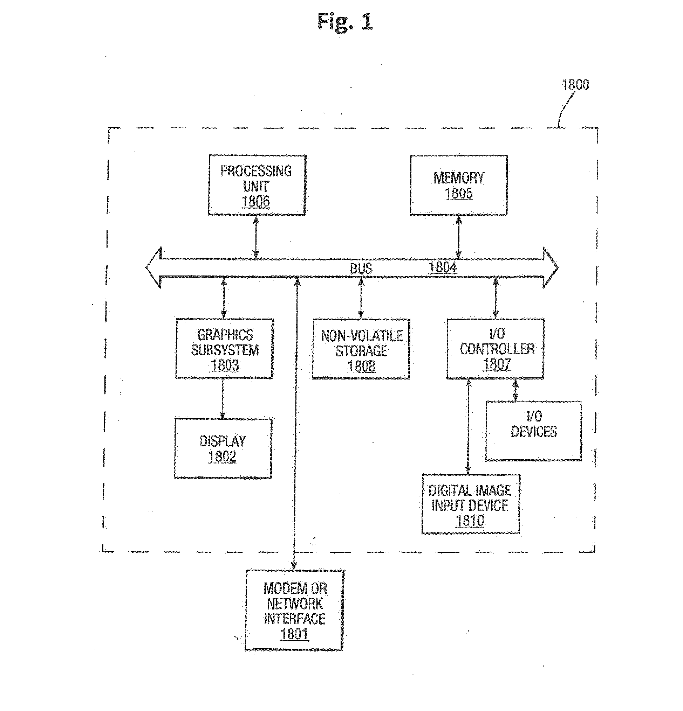

Fig. 1 illustrates an exemplary computer architecture 1800 by which a device embodying or forming part of the personalised medicine system according to the present invention may be implemented. Computer architecture 1800 may be or form part of a desktop computer or a laptop computer, a server, a mobile communications device or any similar computer device.

The computer architecture 1800 may interface to external devices such as another computer, the cloud or Internet, through a modem or network interface 1801, such as an analogue modem, ISDN modem, cable modem, token ring interface, or satellite transmission interface. The network interface 1801 may also be a standard communications module adapted to communicate with a standard mobile communications network, such as 2G, GSM, GPRS, EDGE, 3G, UTMS, CDMA 2000, HDSPA, LTE, 4G, etc networks; a Bluetooth radio; a Wi-Fi radio; or a combination of any two or more of the foregoing.

As shown in Fig. 1, the computer architecture 1800 includes a processing unit 1806, which may be a conventional microprocessor, such as commonly provided by Intel, ARM or AMD, which are known to one of ordinary skill in the computer art. System memory 1805 is coupled to the processing unit 1806 by a system bus 1804. System memory 1805 may be a DRAM, RAM, static RAM (SRAM) or any combination thereof. Bus 1804 couples processing unit 1806 to system memory 1805, to non-volatile storage 1808, to graphics subsystem 1803 and to input/output (I/O) controller 1807. Graphics subsystem 1803 controls a display device 1802, such as a liquid crystal display device, which may be a touchscreen device and/or may be part of the graphics subsystem 1803. The other I/O devices may include one or more of a keyboard, disk drives, printers, a mouse, a speaker, a camera 1810 and the like as known to one of ordinary skill in the computer art.

In one embodiment, the personalised medicine system device 1 is implemented using an application or a widget loaded onto a mobile communications device (such as a smart phone or tablet computer) having a touch screen display device 1802 and a camera 1810 and a speaker (not shown) as I/O devices, and optionally a microphone (not shown) as a further input device. The application causes the device 1 to store predetermined data in the non-volatile storage 1808, which is used to provide the required functionality.

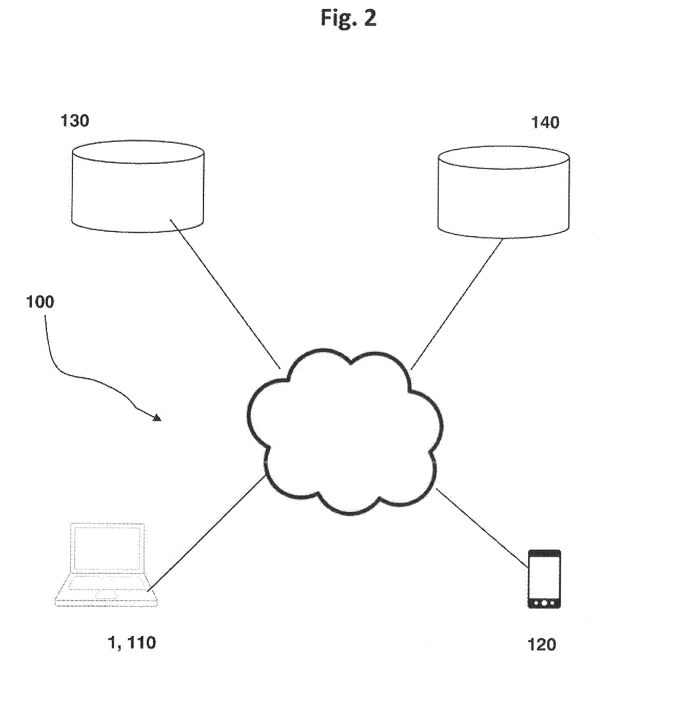

Fig. 2 shows a personalised medicine system 100 comprising a clinician computer 1, 110, a patient smartphone 120, and external servers/computers 130, 140 all of which are connected by one or more of a LAN, a WAN and the Internet. As will be discussed in detail below, the personalised medicine system stores various data, such as workflow information, page information, patient information, slider placement calculation information, side effect severity calculation information, knowledge base information and so forth. Such information may all be stored only on the clinician computer 1, embodying a personalised medicine system of the present invention by itself, or distributed between the clinician computer 110 and one or more networked or remote servers/computers 130, 140, which together embody the personalised medicine system 100 of the present invention. Likewise, processing operations required to provide the functionality of the personalised medicine system 100 may be carried out by the clinician computer 1 alone or may be distributed between the clinician computer 110 and the servers/computers 130, 140. Accordingly, where this specification discusses a particular device such as device 1, 110 storing particular information or carrying out a particular function, it should be understood that in different embodiments the storage and/or functions may be carried out by another device 130, 140 as well or instead.

The patient smartphone, tablet computer or other patient device 120 may provide information to the system 100, such as a record of patient adherence to a treatment regimen, as will be discussed in more detail below.

In some embodiments, multiple clinicians' computers 110 and/or patient devices 120 may be included in the system of the present invention.

*General Operation*

The personalised medicine system 1, 100 operates to guide a clinician through a series of interviews with a patient to assist the clinician to gather information about the patient and to decide on appropriate treatment. Thus, the personalised medicine system 1, 100 acts as a clinical decision support system (CDSS) and provides a personalised patient pathway to tailor diagnosis and treatment of an illness or other medical condition to specific patients. In the following description, the personalised medicine system 1, 100 will be described with reference to schizophrenia as an example of an illness/medical condition to which the personalised medicine system is tailored. However, it should be understood that the personalised medicine system 1, 100 can be tailored to other illnesses/medical conditions such as rheumatoid arthritis and cancer, as well as being applied to illnesses/conditions in general.

In order to carry out this functionality, the personalised medicine system 1, 100 stores a plurality of pages for displaying to a user and one or more workflows, each of the pages being associated with a step or node in a workflow. Each node in the workflow has one, two more pages associated with it.

In the present embodiment, the personalised medicine system 1, 100 stores three workflows, Workflow 1 for a first consultation with a patient, where the patient has not previously reported a contact with a psychiatrist or other mental health professional; Workflow 2 for a first consultation where the patient has previously reported a contact with another psychiatrist or mental health professional; and Workflow 3 for a subsequent consultation using the personalised medicine system 1, 100.

When a user (who will generally be a psychiatrist or other clinician) first starts the personalised medicine system 1, 100, for example by clicking an icon on a desktop or tapping an icon on the touch screen display device 1802 of a tablet computer, or navigating their browser to a specific address on a PC or mobile device, the personalised medicine system 1, 100 first displays a welcome screen (not shown). Here the user is able to select an existing patient previously stored in the system 1, 100 or create a new patient. If the user creates a new patient in the system, he is given the option to select whether or not this is patient's first psychiatric contact. Depending on the choices the user makes at this stage, one of the three respective workflows is selected. Each of the three workflows includes a plurality of stages, comprising one or more steps; one or more nodes associated with each step; and one or more pages associated with each node. One or more knowledge bases may be used to populate various pages. Commencement of a workflow starts a "visit", representing a consultation between the clinician and the patient during which information is reviewed and or recorded in the personalised medicine system 1, 100.

As will be discussed in more detail below, users can also access previous visits and use the workflow to move around the visit to review and/or adjust previously recorded information, as well as to add new information.

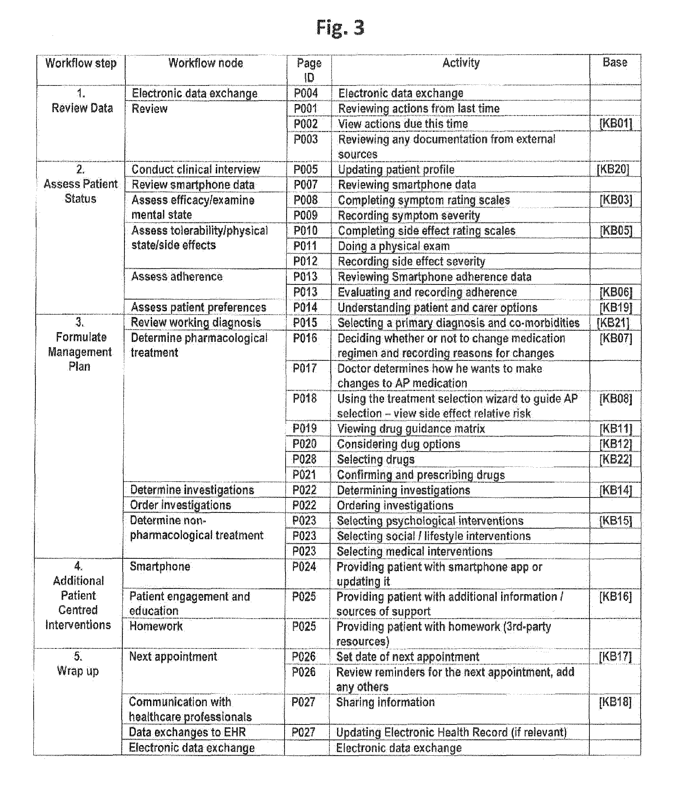

Fig. 3 shows in tabular form Workflow 3 provided for a subsequent consultation by way of example. The other two workflows are similar but changed to take account of their different purposes. The present invention will be described by way of example with reference to the workflow shown in Fig. 3 with relevant changes to the other workflows explained below.

The first workflow step in Fig. 3 for a new consultation with an existing patient is to Review Data and includes two nodes - Electronic Data Exchange and Review. The Electronic Data Exchange node is associated with page ID P004 (not shown). This allows the user to cause the personalised medicine system 1, 100 to interact with other healthcare IT technologies in the clinic/hospital/health authority to carry out automatic data exchange, for example to import patient details stored in electronic health/medical records (EHRs or EMRs) elsewhere, to view previously prescribed and to prescribe drugs, to order lab tests and obtain the results of previously ordered tests. This node may also allow the user to manually enter data, particularly where this is the first psychiatric contact in Workflow 1.

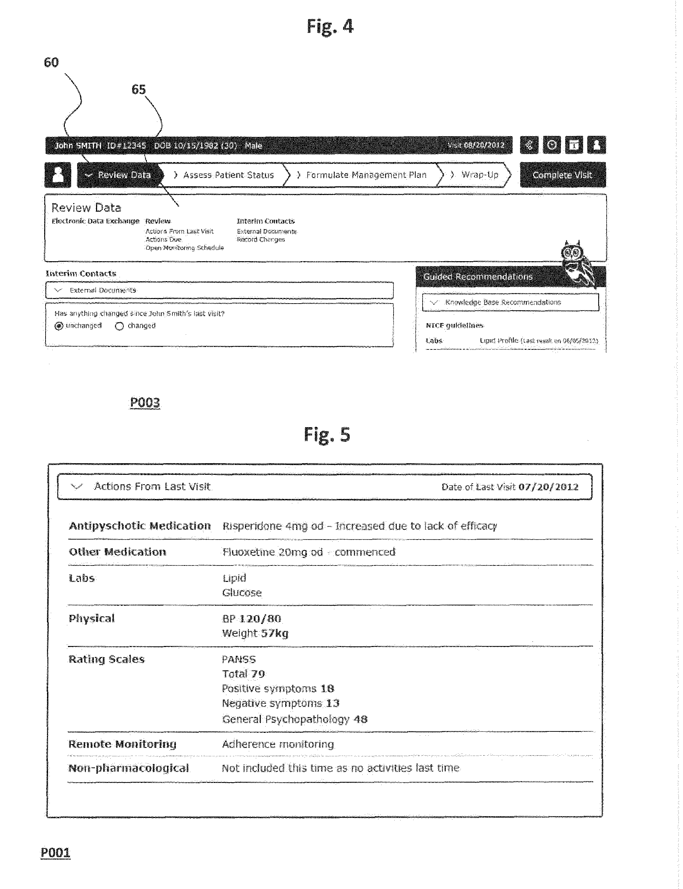

The workflow then moves to the Review node, causing it to display an associated screen. An extract of the screen heading is shown in Fig. 4, which includes the workflow steps in a main heading area 60 and the nodes for the selected step in a sub-heading area 65. The sub-heading area 65 also displays for each node a list of the related activities for which the associated pages are provided. Users can navigate through the workflow by clicking on the respective steps to select them. Once selected, a step is highlighted and a screen sub-heading shows each of the nodes for that step and each of the pages associated with the node. Thus, the user is also able to navigate between nodes and pages by clicking on them to select them. This display structure allows the user to navigate through the workflow in an easy way. Preferably, however, NEXT and PREVIOUS buttons and/or arrows are displayed at the bottom of the screen or elsewhere to allow the user to navigate through a workflow sequentially during a consultation.

In Fig. 4 it can be seen that the user is in the Review Data step of the workflow and is provided with options to enter the Electronic Data Exchange node (discussed above) and the Review node. Within the Review node, the user is able to access pages for reviewing actions from the last visit [page ID P001], viewing actions that are due to be taken this visit [P002] and reviewing documentation from external sources [P003]. In fact, in Fig. 4 the implementation is slightly different from the workflow in Fig. 3 and the option to review documentation from external sources is provided in a separate Interim Contacts node, where it is envisaged that the clinician will view documents created and information changed during an interim contact of the patient with another medical professional since the last consultation. Such interim contacts may occur, for example, because the patient has been admitted to hospital or has obtained psychiatric treatment from another person while on an extended stay at another location. The workflow may be as shown in Fig. 3 or illustrated in Fig. 4.

If the user selects the Review Actions from Last Visit option, for example by clicking on the corresponding wording using a mouse, then page P001 shown in Fig. 5 will be inserted in the screen shown by the display device 1802. Although not shown in Fig. 5, heading area 60 and sub-heading area 65 would also be displayed. Page P001 shows which actions took place as a result of the last visit. Thus, Fig. 5 shows that in the last visit the patient was prescribed risperidone as an antipsychotic medication, fluoxetine as other medication, lipid and glucose labs were ordered, blood pressure and weight were measured, various rating scales were used to assess the patient's symptoms and side effects suffered by the patient, and so forth.

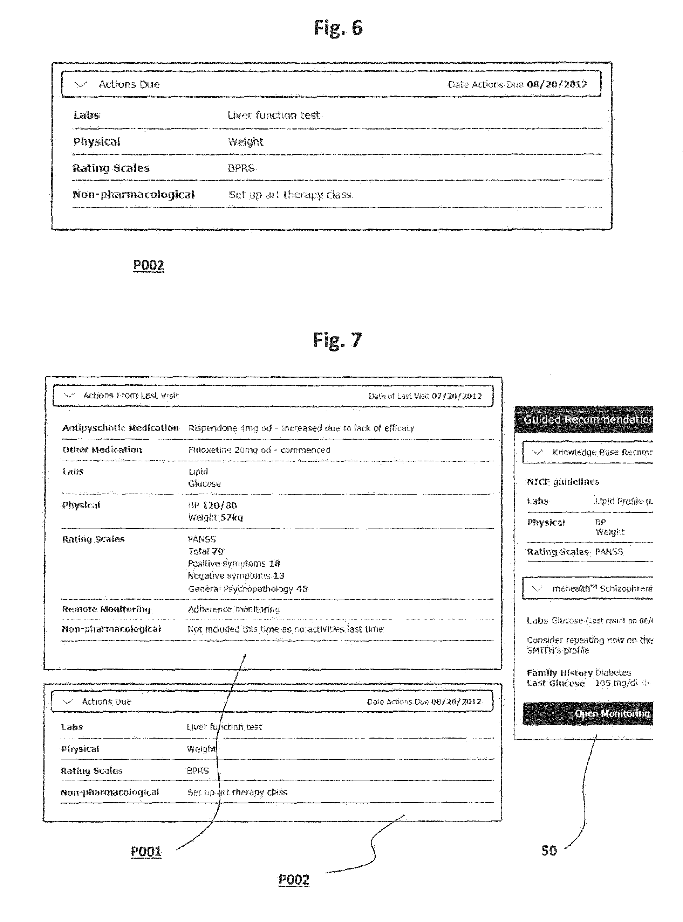

Fig. 6 shows the page P002 by way of further example, which provides the user with a list of actions that should be taken during this consultation. The manner in which this page is populated will be discussed in further detail below.

As illustrated in Fig. 7, in some cases the personalised medicine system 1, 100 may display two or more pages at the same time on the display device 1802. In particular, Fig. 7 shows the concurrent display of pages P001 (actions from last visit) and P002 (actions due). Moreover, as shown in Fig. 7, in addition to displaying the selected pages P001, P002 and P003, the personalised medicine system 1, 100 may simultaneously display guided recommendations for the clinician in guidelines area 50. Again, the population of guidelines area 50 will be discussed in more detail below. As before, heading area 60 and sub-heading area 65 would also be displayed simultaneously.

In workflow 3, following completion of the Review Data step, the workflow moves to the Assess Patient Status step and in particular the first node of conducting a clinical interview. In workflows 1 and 2, since this is the first time the patient has been entered in the personalised medicine system 1, 100, there can be no review of existing data and no due actions will have been set. Accordingly, workflows 1 and 2 omit the Review node of the Review Data step and, following electronic data exchange, move directly to the Assess Patient Status step of the workflow.

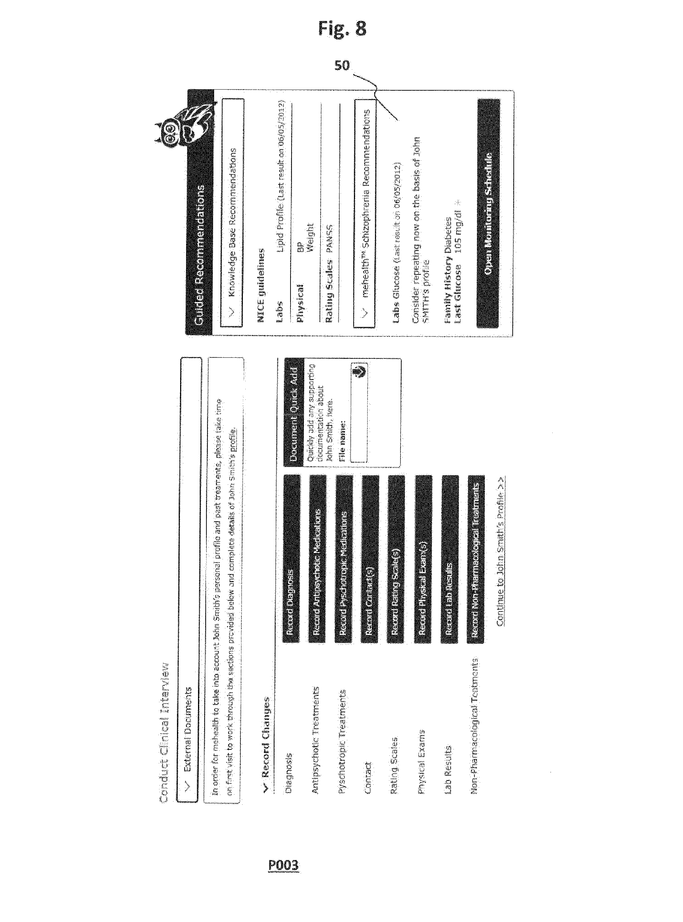

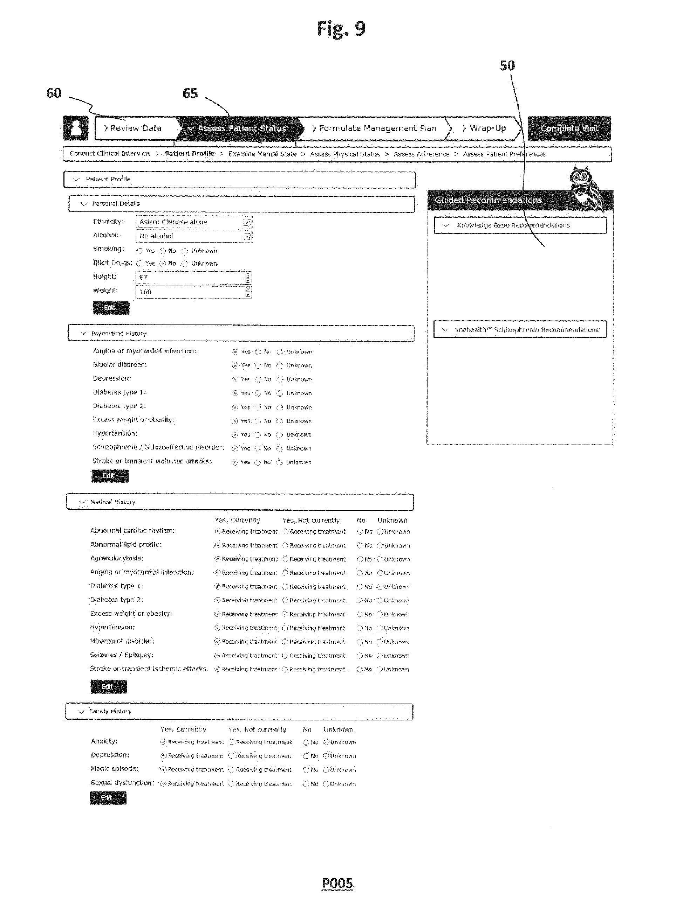

In the Assess Patient Status step of workflows 1 and 2, the first node of conducting a clinical interview is associated with pages P003 (discussed above in respect of workflow 3) and P005. In workflow 3, the conduct clinical interview node is associated only with page P005. Fig. 8 shows an example of page P003 in workflows 1 and 2 together with guidelines area 50. Fig. 9 shows an example of page P005 with guidelines area 50. In workflows 1 and 2, the clinician records the patient profile and clinical history from any available source, including the patient, by systematically working through the prompts in page P003. As shown in Fig. 8, these may include an existing (if any) diagnosis, antipsychotic treatments, other psychotropic treatments, contacts, rating scales, physical examinations, results of laboratory tests, non-pharmacological treatments and so on. Similarly, the clinician completes the patient profile in workflows 1 and 2 or updates the previously-completed patient profile in workflow 3 by following the prompts shown in Fig. 9.

Workflows 1 and 2 then move onto the "examine mental state" node, whereas workflow 3 first allows the clinician to review smartphone data in the "review smartphone" node in conjunction with page P007. In particular, during the (preferably first) visit, the patient's smartphone 120 is updated with an application that allows him to record when he takes his medications and how he is feeling at a given time. The data that has subsequently been input by the patient is then transferred from the smartphone or other patient device 120, for example at predetermined times or during a consultation, onto the clinician's computer 1, 110, and is used to populate page P007 for review by the clinician during the consultation.

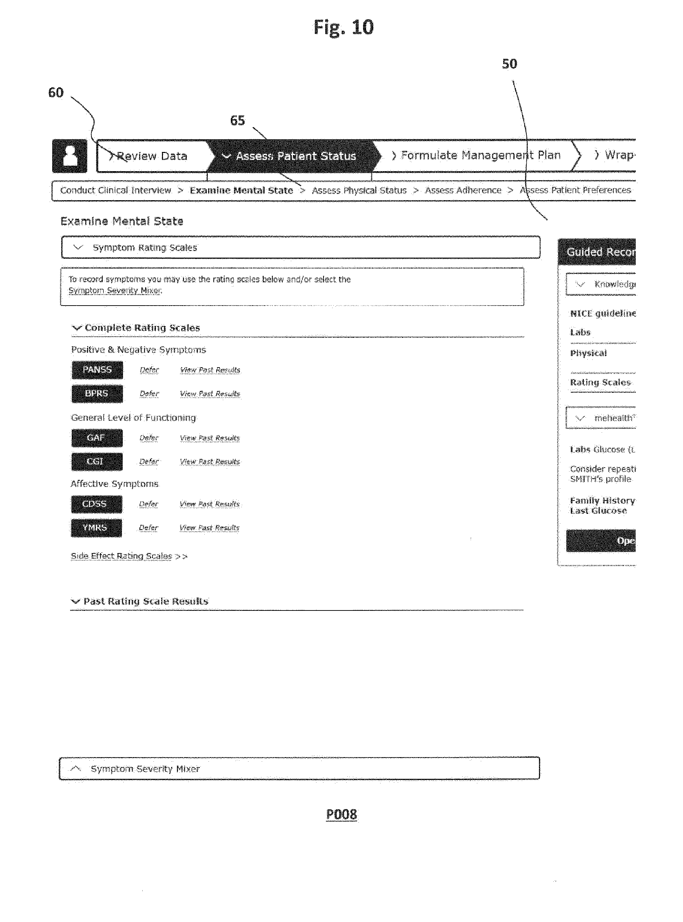

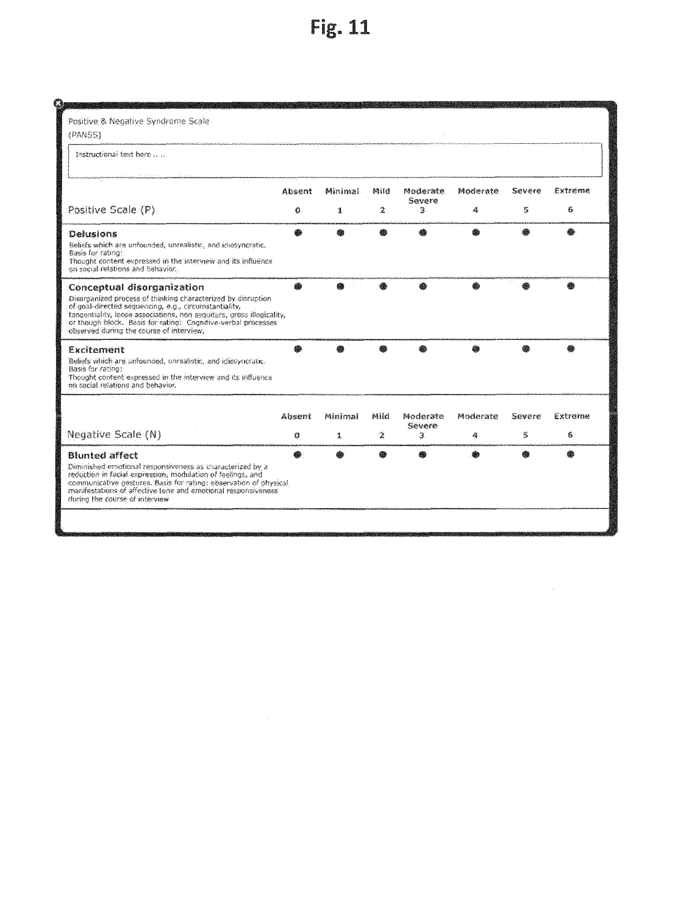

In the "examine mental state" node (which corresponds to the "assess efficacy" node in workflow 3), the user is presented with the option of completing one or more well-established rating scales for assessing the mental state of people suffering psychotic disorders such as schizophrenia. Thus, Fig. 10 shows the display of the screen header 60, 65 highlighting the Assess Patient Status step in the workflow and the "examine mental state" node together with the guidelines area 50. The exemplary symptoms ratings scales shown in Fig. 10 are the positive and negative syndrome scale (PANSS), brief psychiatric rating scale (BPRS), global assessment of functioning (GAF) scale, clinical global impression (CGI) scale, Calgary depression scale for schizophrenia (CDSS) and young mania rating scale (YMRS). However, any number of rating scales may be provided and the user can select any one of the available scales by clicking the corresponding icon to cause a pop-window to open, for example as shown in Fig. 11. The user can then complete the rating scale.

For example, the PANSS is a medical scale used for measuring symptom severity of patients with schizophrenia. To assess a patient using PANSS, an approximately 45-minute clinical interview is conducted. The patient is rated from 1 to 7 on 30 different symptoms based on the interview as well as reports of family members or primary care hospital workers. The symptoms are classified into a positive scale, a negative scale and a general psychopathology scale. The positive sub-scale includes 7 items, minimum score = 7, maximum score = 49 (delusions, conceptual disorganization, hallucinations, hyperactivity, grandiosity, suspiciousness/persecution and hostility); the negative sub-scale includes 7 items, minimum score = 7, maximum score = 49 (blunted affect, emotional withdrawal, poor rapport, passive/apathetic social withdrawal, difficulty in abstract thinking, lack of spontaneity and flow of conversation, and stereotyped thinking) and the general psychopathology sub-scale includes 16 items, minimum score = 16, maximum score = 112 (somatic concern, anxiety, guilt feelings, tension, mannerisms and posturing, depression, motor retardation, uncooperativeness, unusual thought content, disorientation, poor attention, lack of judgment and insight, disturbance of volition, poor impulse control, preoccupation, and active social avoidance).

The other scales operate in a similar manner but include different numbers of symptoms and different scales. For example, the GAF scale is simply a single number selected by the clinician based on his assessment of the patient's overall functioning, based on guidance the scale provides for different number ranges within the scale. Although the different scales may test for different things, generally there is some overlap between scales and different scales may test for (some of) the same things in different ways, for example with different questions and/or different numbers of questions. Other scales may also assess the patient's well-being in different non-clinical domains, for example, quality of life.

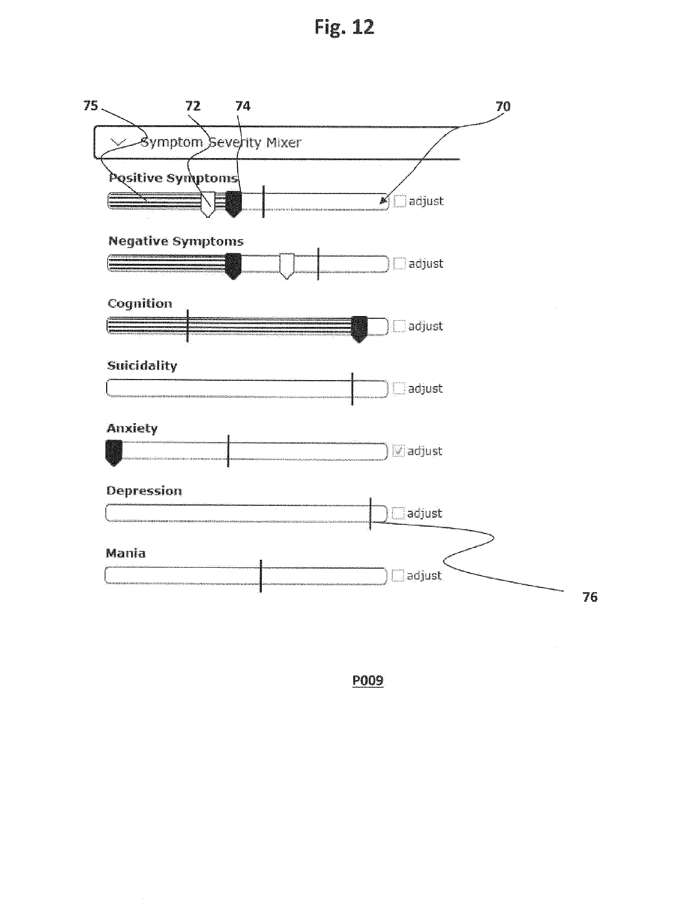

Based on the completion of one or more scales (or none) by the clinician, the personalised medicine system 1, 100 calculates a symptom severity value for each of a plurality of predetermined symptoms and displays the results of the calculations on a graphical scale for each of the symptoms on page P009, as illustrated in Fig. 12. In particular, page P009 shows a graphical representation of linear scale 70 for each of positive symptoms, negative symptoms, cognition, suicidality, anxiety, depression, and mania. Although not illustrated, other scales could also be provided, for example quality of life, and general level of functioning, although this is not an exhaustive list. These graphical representations may be termed symptom (severity) sliders 70 or mixers. In addition to populating the sliders 70 based on the clinician's data input to one or more rating scales in page P008, the user is able to enter a value on the scale based on his own impressions or additional information gained during the visit. Thus, in Fig. 12 shows for each slider 70 a light icon 72 representing a normalised value calculated by the personalised medicine system 1, 100 based on completion of one or more rating scales; a dark icon 74 representing a value enter by the clinician; a fill 75 from 0 up to the dark icon 74 to indicate clearly that the most important value is the clinician updated value; and a line 76 representing the previous value set during the previous visit (which corresponds to the previously set clinician icon 74 or, if the clinician did not input this, the previously calculated normalised value based on completion of one or more rating scales).

Preferably, the user enters a value manually by clicking on the selected slider and dragging an icon along the scale to the appropriate position. If a value has already been calculated by the personalised medicine system 1, 100 or a previous value has been stored, this will be displayed on the slider and the user will be able to use this information together with his experience to position the manually input slider. Alternatively the user may be able to enter a value in box (preferably from 0-10 or 0-100) and this will position the manually input slider on the graphical scale.

The symptom sliders will be discussed in more detail below.

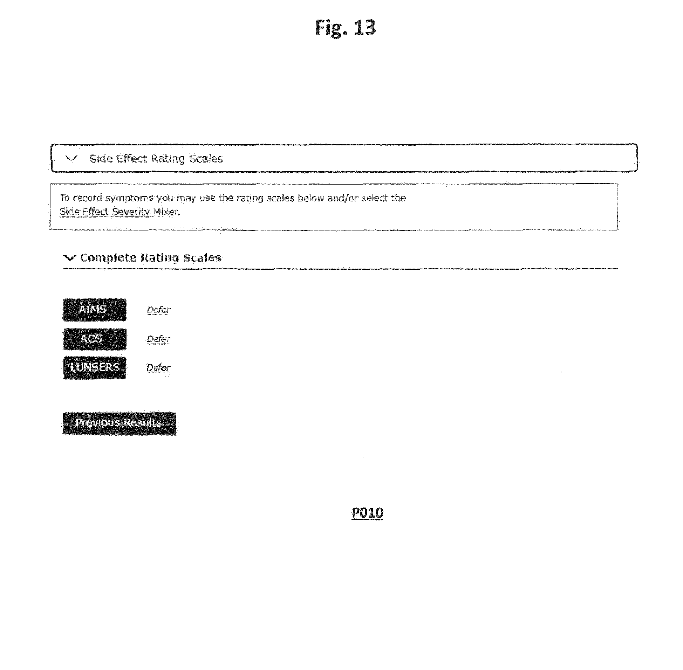

Following completion of the "examine mental state"/"assess efficacy" node, the workflow then moves on to the "assess physical status/side effects"/"assess tolerability" node. This node is associated with page P010, shown in Fig. 13. In this case, the user is given the option to complete one or more predetermined ratings scales to assess side effects of medications experienced by the patient. In the example, the scales are the abnormal involuntary movement scale (AIMS), the antipsychotic side-effect checklist (ASC) and the Liverpool University neuroleptic side effect rating scale (LUNSERS). Again, any number of other rating scales may be provided and again clicking one of the icons causes a pop-up window to open to guide the user through completion of the respective scale.

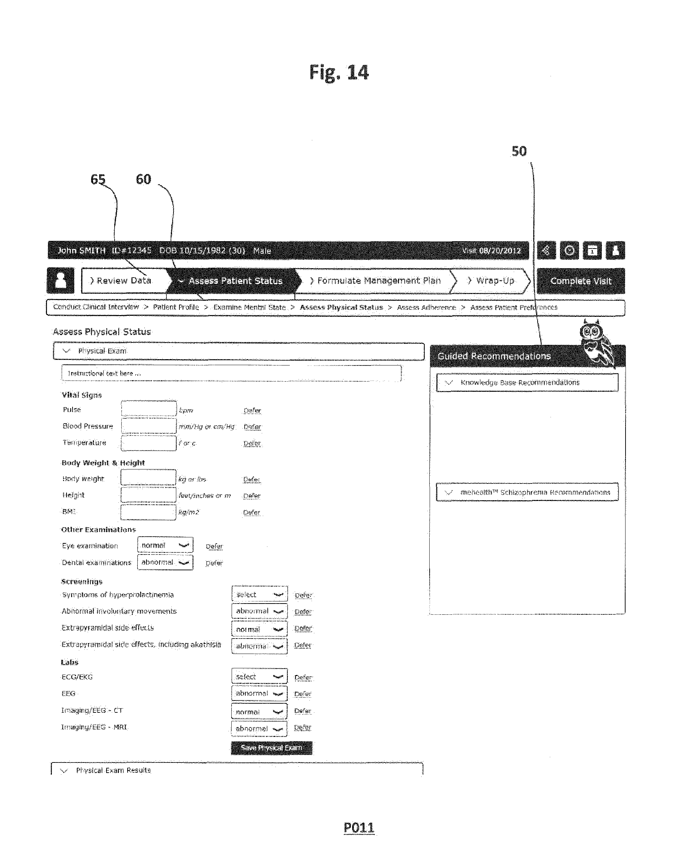

The next page associated with the node is the physical exam page P011. Fig. 14 shows an example of page P011 together with the relevant screen heading 60 and sub-heading 65, as well as the guidelines area 50. As with the other pages, alternative examples are possible. In any event, the user systematically follows the options presented on the page to enter the respective patient details such as height, weight, blood pressure and so forth.

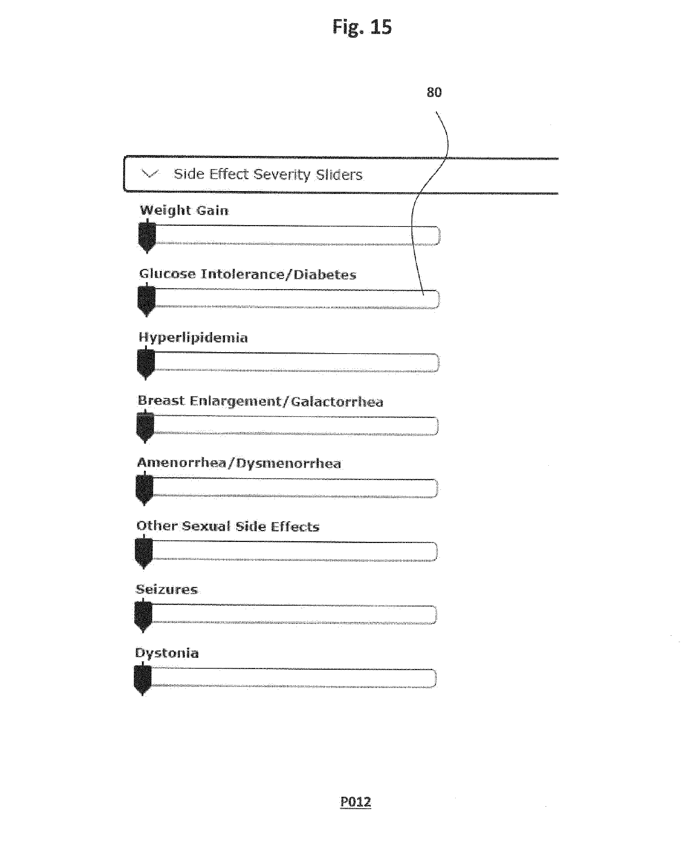

Following completion of pages P010 and P011, the personalised medicine system 1, 100 proceeds to display page P012, shown in Fig. 15, showing side effect (severity) sliders 80. These are populated based upon the completed side effect rating scales and, preferably, the input physical examination data. Similarly to the symptom severity sliders discussed above, the side effect severity sliders include a light icon representing a normalised value calculated by the personalised medicine system 1, 100; a dark icon representing a value manually entered by the clinician; a fill from zero up to the dark icon to indicate clearly that the most important value is the clinician updated value; and a line through the scale representing the previous value set during the previous visit (which corresponds to the previously set clinician icon or, if the clinician did not input this, the previously calculated normalised value based on completion of one or more side effect rating scales).

The user manually enters a value in a side effect severity slider in the same way as discussed above in respect of the symptom severity sliders.

The side effect sliders will be discussed in more detail below.

The workflow then moves on to the "assess adherence" node to allow the user to assess how well the patient has adhered to previous or on-going treatment, for example by reviewing data from the patient's smartphone 120 and by checking boxes and entering other information as guided by the corresponding page(s). This node may be omitted or significantly altered in workflows 1 and 2, for example, if the patient is not currently prescribed any medication.

Finally in the Assess Patient Status step, the workflow moves on to the "assess patient preferences" node, where the clinician and patient decide whether to continue with existing medication or to change medication. If a decision to change medication is taken, a pop-up window may open giving the user the option to enter reasons for the change, such as partial efficacy, lack of efficacy or poor tolerability. Again, this node may be omitted or significantly altered in workflows 1 and 2.

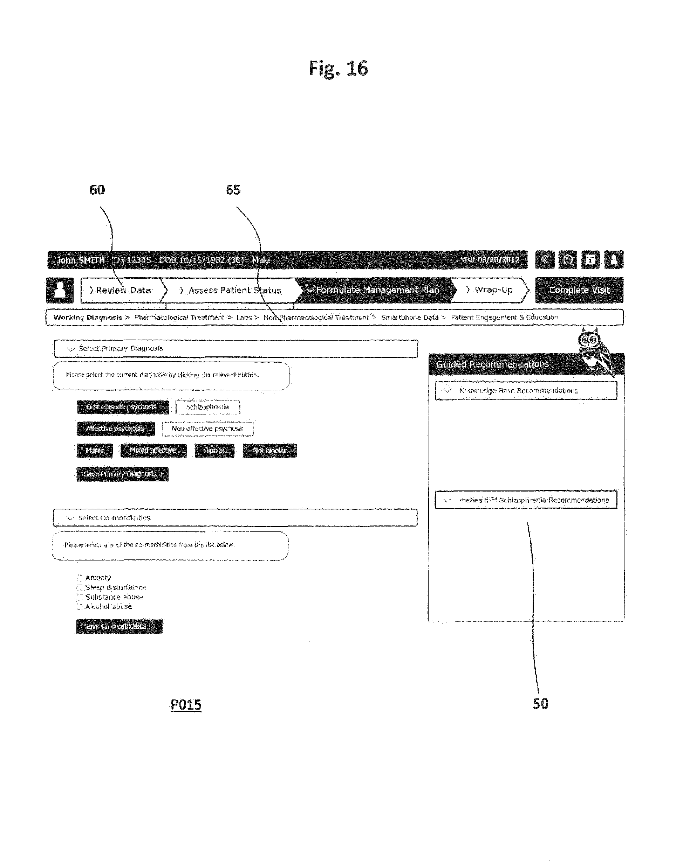

The workflow then moves on to the Formulate Management Plan step. In the first node, "review working diagnosis", page P015 is displayed, as shown in Fig. 16, allowing the user to select or confirm a primary diagnosis and co-morbidities. Preferably, the display of page P015 may change as the primary diagnosis is entered. For example, it may first offer the option of selecting first episode psychosis (FEP) or schizophrenia as the primary diagnosis. If FEP is selected, the page may then offer the selection of affective psychosis or non-affective psychosis. If affective psychosis is selected, the page may then offer the selection of manic, mixed effective, bipolar and non-bipolar. The page also allows the selection of co-morbidities, for example, anxiety, sleep disturbance, drug abuse and alcohol abuse.

In workflows 1 and 2, a "select primary diagnosis" or "determine working diagnosis" node substitute for the "review working diagnosis" node.

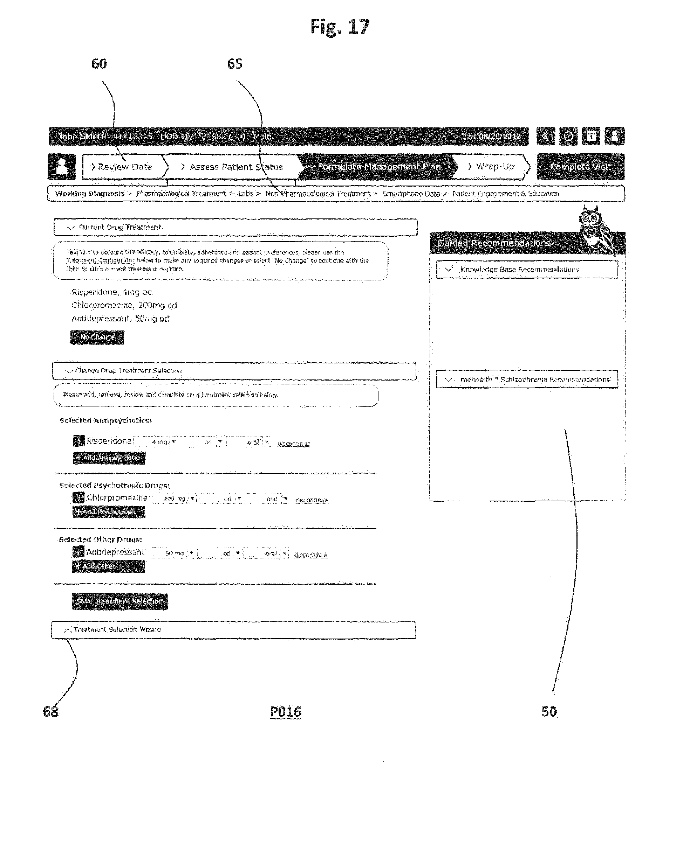

The workflow then moves on to the "determine pharmacological treatment" node, involving the sequential display of pages P016, P017, P018, P019, P020, P028 and P021. Page P016 is shown in Fig. 17 together with heading 60, sub-heading 65 and guidelines area 50. In page P016 the user is shown the current drug treatment regimen and the ability to change the regimen. If the user decides to make a change, pop-up window P017 (not shown) opens requesting the user to select one or more reasons for the decision. As shown in Fig. 17, the user may select one or more new drugs in page P016.

If the user decides not to make any changes, the workflow proceeds to show page P020. In the screen shown on display device 1802, the personalised medicine system 1, 100 provides an option of using an antipsychotic (AP) drug selection wizard or routine provided by the personalised medicine system 1, 100 (see section 68 in Fig. 17) at the same time as showing page P016. If the user decides not to use the wizard, the workflow accepts the changes made in page P016. Otherwise, the workflow moves to page P018.

It will be appreciated that instead of allowing the user to make changes to the drug selection in page P016, an intermediate page may be provided to allow the user either to use the personalised medicine system 1, 100 wizard or to manually select the AP drugs on a new page, which would be similar to the "change drug treatment selection" part shown at the bottom of page P016.

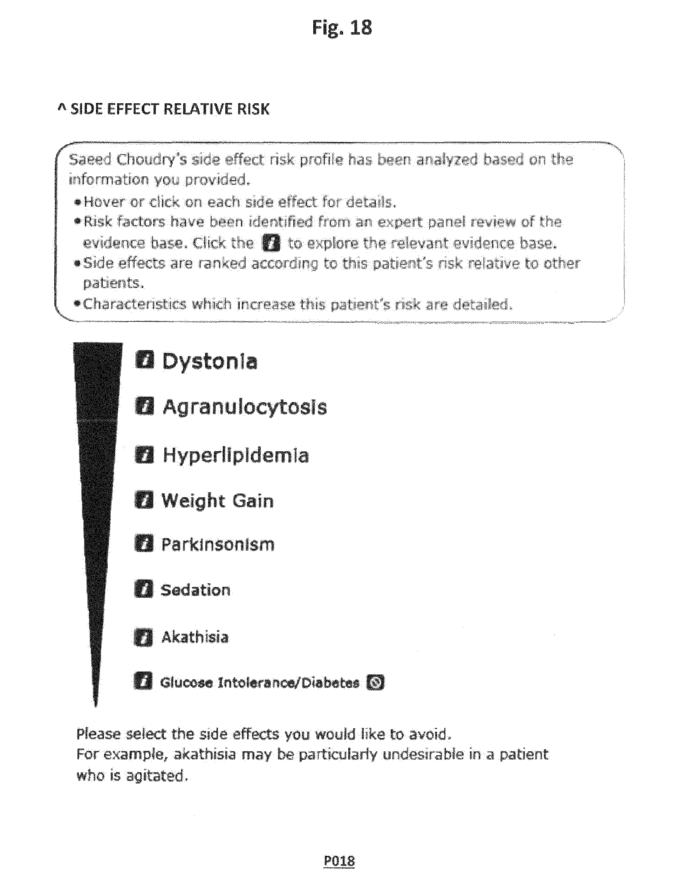

If the user decides to use the wizard, the personalised medicine system 1, 100 may first present, for a plurality of potential side effects, any characteristics which the patient possesses which increase the likelihood of experiencing the side effect. The strength of influence on the side effect of each specific characteristic is presented. The personalised medicine system 1, 100 then calculates, for a plurality of potential side effects, a risk that the patient will experience the side effect relative to the general population. The side effects are then shown in ranked order in page P018, as illustrated in Fig. 18. The manner in which the relative side effect risks and ranked order are determined will be discussed in more detail.

The user may optionally select one or more of the listed side effects which the clinician or patient wants to avoid. For example, a particular patient may particularly want to avoid weight gain or a clinician may recognise that akathisia is undesirable in a patient who is already agitated. A selected side effect may be shown in a different colour, size or font to the other side effects shown on the page.

The user may also hover over or click on each side effect for further details of the side effect, general factors which moderate the side effect, and specific factors extracted from the patient data used in the calculation. The user may also click on an information button next to each side effect to explore the evidence base for the side effect.

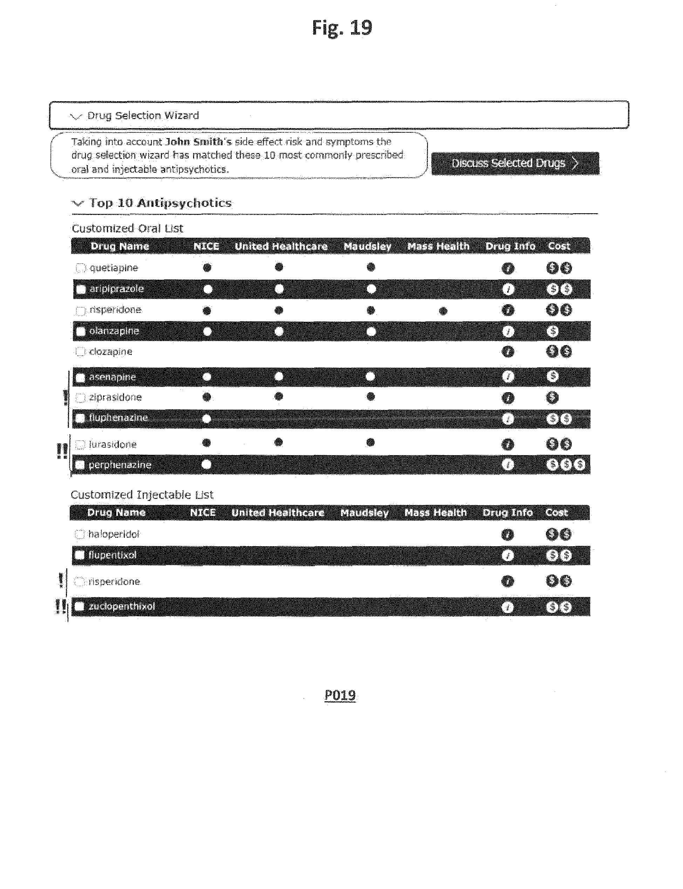

Depending on the working diagnosis selected in page P015, the personalised medicine system 1, 100 then displays in page P019 a list of AP drugs which could be used to treat the patient (see Fig. 19). Moreover, as shown in the figure, the personalised medicine system 1, 100 displays the list of selected AP drugs together with an indication for each drug of whether it is recommended/approved in respective predetermined clinical guidelines by placing a column for each guideline alongside the list of drugs and placing a dot or other marker in each column for each recommended/approved drug. In the example in Fig. 19, the predetermined guidelines are those published by the UK National Institute for Health and Clinical Excellence (NICE), UnitedHealthcare in the US, the Maudsley psychiatric hospital in the UK, and MassHealth in the US. Naturally, these guidelines are shown by way of illustration only and any other guidelines could be chosen as well or instead.

A column is also provided to allow the user to click on an icon for each drug to obtain further information about the drug, for example from one or more of the guidelines, the manufacturer or other sources. Optionally, the user can click on a dot or other icon under the guideline columns to view information in the respective guideline about the respective drug.

A further cost column is provided showing the relative cost of each drug. Again, clicking on an icon in this column may show the user further information about the costs of the respective drug.

Page P019 further includes an indicator (shown on the left hand side in Fig. 19) of the relative risks of each of the displayed drugs to the particular patient, depending on the side effect relative risks calculated by the personalised medicine system 1, 100, a side effect selected to be avoided in page P018 and, optionally, other patient data. In particular, in the example in Fig. 19, ten drugs to be taken orally are displayed with a single exclamation mark (!) placed against drugs 6-8 in the list and two exclamation marks (!!) placed against drugs 9 and 10. Similarly, five drugs to be taken by injection are displayed with a single exclamation mark (!) placed against drug 4 in the list and two exclamation marks (!!) placed against drug 5. A single exclamation mark may be used, for example, to indicate that the drug should be used with some caution, as the patient's profile suggests that he may have an increased risk of certain adverse side effects. Two exclamation marks may be used to indicate that the drug should be used with extreme caution, as the patient's profile suggests that he is likely to have an increased risk of adverse side effects. Clicking on an icon adjacent the drug may provide further information about what the adverse side effects are and the relative risk of experiencing them. The number of exclamation marks also takes into account the views of the patient and doctor about which side effects would be most undesirable.

Accordingly, the drug table provides a wealth of information tailored to the specific patient to allow the clinician and the patient to discuss suitable AP drug treatment options and select an AP drug for treatment.

Irrespective of whether the pharmacological treatment has been changed, the personalised medicine system 1, 100 then shows page P020 (not shown) which is populated with information about the selected AP drug, allowing further discussion of the treatment options. Subsequently, page P028 (not shown) is displayed, allowing the user to select non-antipsychotic psychotropic medications in addition to the selected AP drug. Optionally, page P028 is only displayed the first time an AP drug has been selected/prescribed or if the AP drug selection has changed. The workflow then moves on to shown page P021 (not shown), in which the user confirms and prescribes the selected drug(s).

The workflow then proceeds to the "determine investigations" node to determine which investigations should be carried out on the patient based on the prescribed drug(s), the patient's status, and other information (such as the evidence base and the guideline recommendations, which are stored in a knowledge base 260 discussed below), to obtain baseline values or follow-up evaluations of the patient's tolerability and physical health status; the "ordering investigations" node to order the determined investigations; the "selecting psychological interventions" node for selecting and ordering interventions such as family therapy, cognitive behavioural therapy (CBT) etc; the "selecting social/lifestyle interventions" node for selecting and ordering interventions such as smoking cessation support, gym referral, supported employment etc; and the "selecting medical interventions" node for selecting and ordering interventions such as dietary advice, alcohol/drugs therapy, diabetologist referral etc.

Finally, the workflow moves on to the Additional Patient Centred Interventions and Wrap-up steps of the workflow with their associated nodes and pages.

Accordingly, it will be appreciated that the personalised medicine system 1, 100 of the present embodiment provides a platform written specifically for clinicians and patients in the area of psychiatry, which addresses the unique needs of practitioners as they relate to patient tracking, clinical decision support, clinical references and guidelines, evaluation (treatment and outcome assessment), patient-facing support, and personalised remote self-monitoring. The personalised medicine system 1, 100 provides personalised best practice clinical management decisions by combining industry-recognised guidelines, a published evidence base, a clear workflow for the clinician to follow and the patient's unique characteristics. The present invention improves the care of individuals with chronic mental illness, where there is a large number of different treatments available, each with unpredictable efficacy and troublesome side effects. In addition to providing guidance on the selection of appropriate drugs for treatment of the patient, the personalised medicine system 1, 100 of the present invention also helps the doctor with making non-drug treatment decisions; obtaining, providing and reviewing patient information; monitoring, recording and presenting outcome etc.

Although the present embodiment relates to a personalised medicine system 1, 100 specifically adapted to the treatment of mental illness and, specifically, schizophrenia and other psychosis, it will be apparent that the personalised medicine system 1, 100 of the present invention can be adapted to a host of other illnesses, including especially those illnesses and conditions that require long term care and multiple consultations between patient and physician, such as various different forms of cancer, rheumatoid arthritis and so on. However, the personalised medicine system 1, 100 of the present invention can also be adapted to provide a workflow for the general practitioner or trained nurse to follow in more general consultations in less specialised fields of practice.

In each case, the personalised medicine system 1, 100 of the present invention can provide point-of-care decision support for a wide spectrum of clinical decisions from diagnosis to chronic disease management. It accompanies the doctor and patient across the full continuum of care and throughout the illness, informing therapeutic decisions, monitoring outcomes and offering patient-focused interventions. In doing so, the personalised medicine system 1, 100 allows clinicians to apply the evidence-base to each individual patient and facilitates concordance of the treatment with selected guidelines, promoting consistently higher quality care. Preferably, it can be customised by healthcare providers and doctors to preferred guidelines or formularies.

Specific aspects of the personalised medicine system 1, 100 will now be described in more detail.

*Context Specific Information*

As previously discussed the personalised medicine system 1, 100 may display on screen, together with the page(s) associated with the relevant node of a step in the workflow, a guidelines area 50. In addition, as shown in Fig. 6, page P003 shows actions due in the current visit. Other such prompts may appear elsewhere in the workflow. Together, the guidelines area 50 and the other prompts due provide the user with context specific advisory information for the user to follow or review as desired. The provision of the context specific advisory information (guidelines area 50 and the actions due prompts) will now be discussed in more detail.

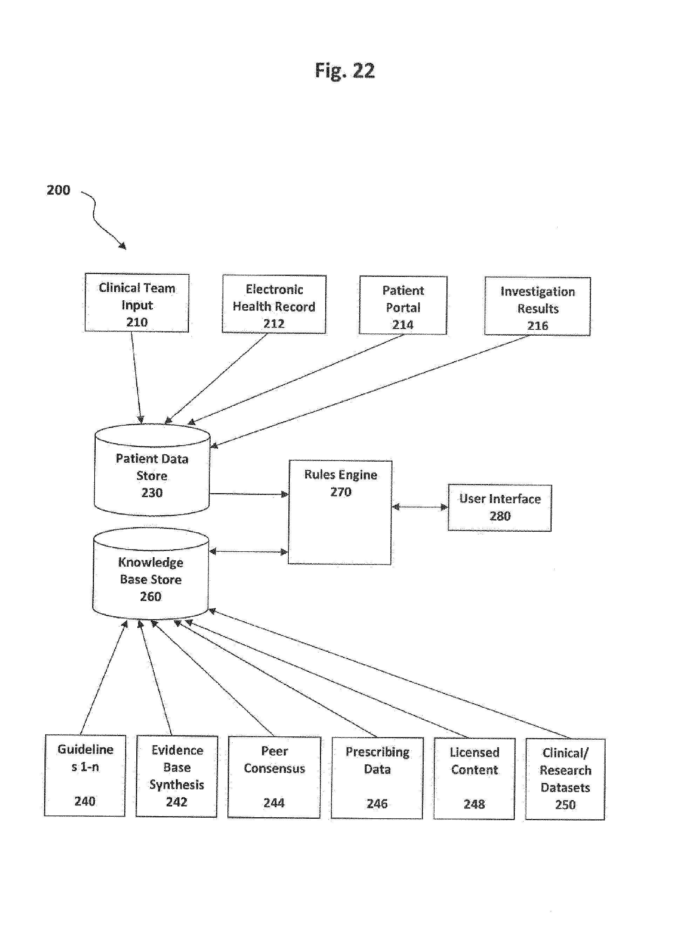

Fig. 22 is a schematic diagram of the advisory information sub-system 200 contributing to the advisory information functionality of the personalised medicine system 1, 100. Specifically, advisory information sub-system 200 comprises a patient data store 230 and a knowledge base store 260.

The patient data store 230 is populated for each patient with information about that patient derived from various sources, such as clinical team input 210; existing electronic health/medical records (EHR/EMR) for the patient 212; a patient portal 214 which receives inputs from the patient in clinic or via their mobile communications or other device 120 (eg smartphone or tablet) for example concerning their mood or taking of medications (including remote monitoring of the patient); and investigation results 216 for example from labs or results of personalised medicine tests, for example for genetic markers.

The knowledge base store 260 derives from inputs from various sources, including various different published clinical guidelines and treatment algorithms 240 (for example, from NICE, UnitedHealthcare and MassHealth); a synthesis of the evidence base 242 relating to the condition to which the personalised medicine system 1, 100 is tailored (for example, mental illness); peer consensus 244; predetermined prescribing data 246; licensed content (for example, a clinical textbook such as the Maudsley Prescribing Guidelines) 248; and clinical/research datasets 250 including de-identified patient data from the system. Known knowledge capture and data mining processes may be carried out on these sources to extract relevant information and to populate the knowledge base. Such processes may be carried out automatically using a computer program to parse and interrogate each knowledge source or by a human, or more usually by a combination of automatic and manual operations.

For example, US patent number US 6,868,422 discloses a method of knowledge acquisition in expert systems suitable for use in the present invention in which data is collected into a computer entity by compiling a set of knowledge data statements, each knowledge data statement representing an element of a knowledge domain; creating lattice data comprising a plurality of nodes with connections between the nodes, each node representing a knowledge data statement, the nodes being arranged in a hierarchical structure and the connections comprising logical connectivity relationships; and creating a consistency matrix, which comprises an array of data entries representing consistency relationships between the knowledge data statements. The data statements can be elicited by using a questioning structure determined from an underlying mathematical base. The contents of US 6,868,422 are incorporated herein by reference.

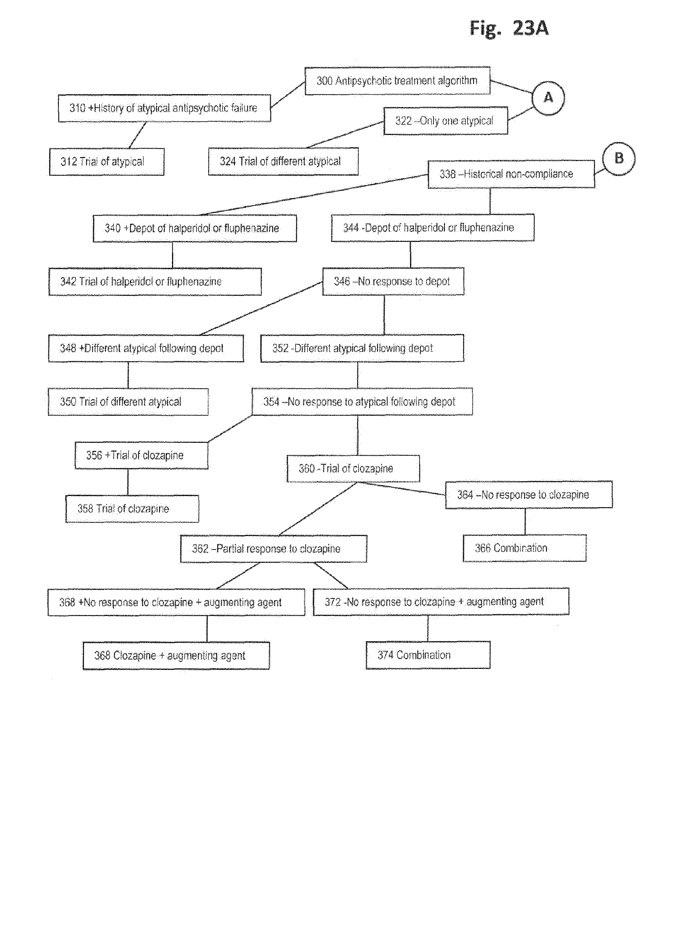

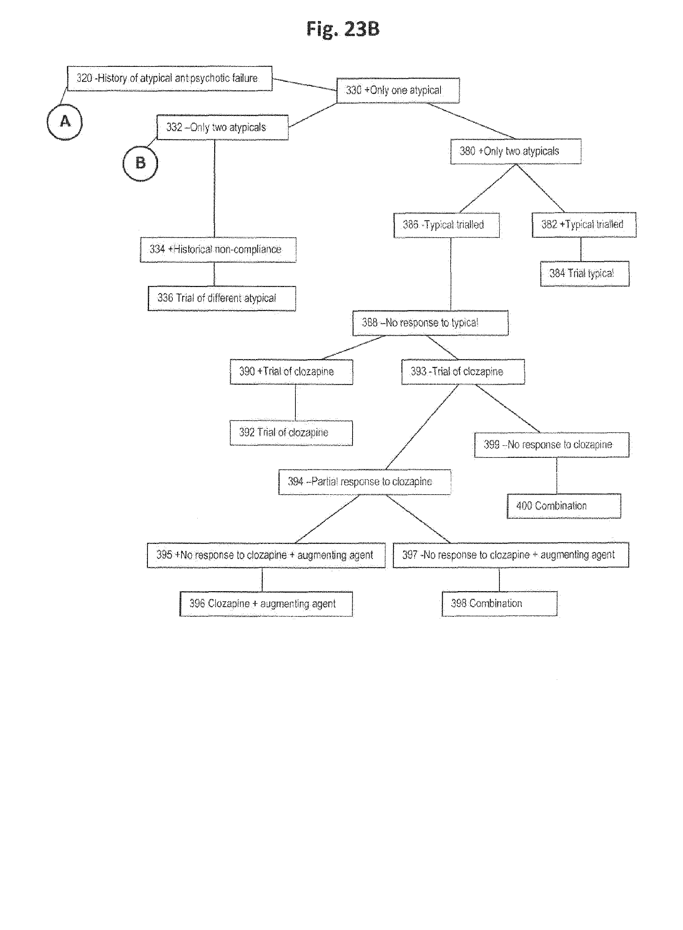

The knowledge base store 260 further stores a plurality of rules for providing the relevant information to the user interface 280 at the relevant time. An example of such a rule is shown in Fig. 23, which provides an antipsychotic treatment rule. The rule comprises a number of knowledge statements, each representing a node in a hierarchical structure with connections between the nodes representing consistency logical relationships between the nodes. In Fig. 23, the root node 300 has two branches leading to nodes 310 and 320, which query whether there has been a history of failure of treatment by atypical antipsychotic (AP) drugs. If there has been no history of treatment failure the network is followed to node 310 and then to node 312, which recommends trialling an atypical AP drug.

By contrast, if there has been a history of failure, the network is followed to node 320 and from there to node 322 if only one atypical AP drug has previously been tried, in which case node 324 suggests trialling a different atypical AP drug.

By contrast, if more than one atypical AP drug has previously been tried, the network is followed to node 330 and from there to node 332 if only two atypical drugs have previously been tried. From node 332 the network is followed to node 334 if there has been no history of non-compliance and to node 338 if there has been a history of non-compliance. If the network is followed to node 334, a recommendation is given in node 336 to trial a different atypical drug.

From node 338 the network is followed to node 340 if a depot neither haloperidol nor fluphenazine have previously been tried, in which case a trial of a depot of one of them is recommended at node 342. If a depot of one of them has previously been tried (node 344) and there has been no response to the depot (node 346), then it is queried whether a different atypical drug has been tried following the depot. If not (node 348), then trial of a different atypical drug is suggested (node 350). If a different atypical drug has been tried following the depot (node 352) and there has been no response (node 354), then it is queried whether clozapine has been tried. If clozapine has not been tried (node 356), the trial of clozapine is recommended (node 358). By contrast, if clozapine has previously been tried (node 360), and there has been a partial response (node 362) and there has not been no response to clozapine with an augmenting agent (node 368), then clozapine with an augmenting agent is suggested (node 370). By contrast, if there has been no response to clozapine with an augmenting agent (node 372), then a combination of clozapine with another atypical AP drug is recommended (node 374). Alternatively, if clozapine has been tried (node 360) with no response (node 364), then a combination of clozapine with another atypical drug is recommended (node 366).

It is noted that in several instances only a node for "no response" is provided and there is no node for "there has been a response"; or only nodes for "no response" and "partial response" are provided. This is because it is assumed that if there has been a response the drug is working well and there will be no desire to change the pharmacological treatment.

Returning to node 330 (not only one atypical AP drug has been tried), the network is followed to node 380 if not only two atypical AP drugs have been tried. The network is then followed in a similar manner down the various branches until a recommendation is reached at the end of a branch.

This rule would be activated to populate the guidelines area 50 shown alongside page P016 (see Fig. 17), for example.

Naturally, the knowledge base store 260 stores a large number of such rules, together with the underlying data to display in the guidelines area 50 once a rule network was been parsed. Such rules may allow for or even require the input of various different items of patient data or other information to be successfully parsed.

These rules may be grouped into subsidiary knowledge bases, and the various pages may call upon the subsidiary knowledge bases to populate the guidelines area 50 or the advisory action section of the page. Thus, the table in Fig. 3 shows that page P002 in the "review" node of the Review Data step in workflow 3 calls upon subsidiary knowledge base KB01, page P005 in workflow 3 calls upon subsidiary knowledge base KB20, and so on.

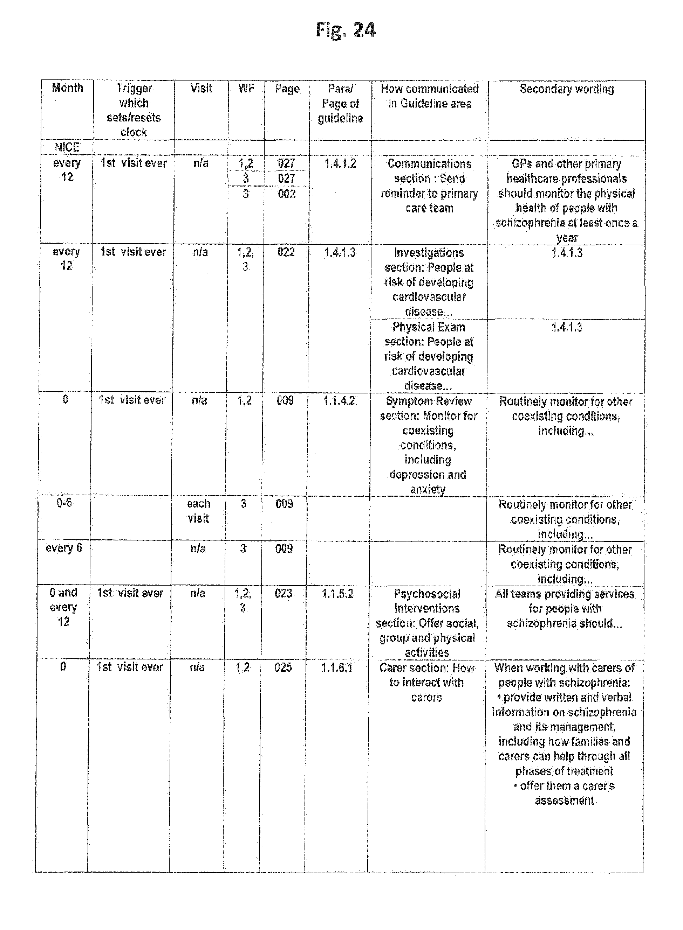

As well as including the subsidiary knowledge bases, the knowledge base store 260 also stores a monitoring rules table, an exemplary extract of which is shown in Fig. 24. In particular, Fig. 24 shows some of the monitoring rules provided for the NICE clinical guidelines included in the knowledge base. However, many more monitoring rules may in practice be provided for this guideline, and an additional set of monitoring rules may be provided for each different guideline. In effect, each guideline is automatically or manually parsed to find each time-based advisory action or item of information and this is incorporated in the monitoring rules table. The monitoring rules table includes columns for a frequency (month - although another unit of time could be selected such as days or weeks); a trigger which sets a clock; a visit; a workflow WF; a page ID; the relevant paragraph or page of the guideline, how the information is communicated to the user in the guidelines area 50; and secondary wording to display on receipt of a request by the user for more information.

For example, taking the first rule in the NICE monitoring table for specific advisory information, the advisory information should be displayed every 12 months starting from the first visit ever. Thus, the information will be displayed in the first consultation between the clinician and patient and the next visit occurring after 12 months has expired. If the visit causes the personalised medicine system 1, 100 to run workflows 1 or 2, the advice is included in the guidelines area 50 when page P027 is displayed. More particularly, the wording "Send primary reminder to care team" is displayed in the Communications section of the page. If the user clicks on this wording or another information icon, the additional wording "GPs and other primary healthcare professionals should monitor the physical health of people with schizophrenia at least once a year", and the user will also have access to the whole NICE guidelines document with the specific wording highlighted.

If the visit causes the personalised medicine system 1, 100 to run workflow 3, the advisory information is included in the guidelines area 50 both when pages P002 and P027 are displayed.

In the second rule, wording is displayed in each of the Investigations section and the Physical Exam section of the "determine investigations" page P022 in each of workflows 1, 2 and 3. If further information is requested, the wording of the appropriate paragraph of the guideline is repeated. As with other pages, it will be appreciated that rules in the knowledge base store 260 other than those included in the monitoring rules table may be used to populate this page, for example based on the drug prescribed for treatment of the patient.

In the third rule, the advisory information is only displayed in the first visit and not in subsequent visits. Accordingly, the rule does not apply to workflow 3.

In the fourth rule, advisory information is displayed each visit for the first six months and is not displayed again afterwards. This rule applies only to workflow 3, so the patient will be on a follow up visit when the advisory information is displayed in page P009.

It will be appreciated that some rules only require the display of particular information in the guidelines area 50 or a predetermined part of a page, without any further conditions are being required. By contrast, other rules may be very complicated and require checking whether a large number of conditions are met before a decision is made on what advisory information, if any, is to be displayed.

The advisory information sub-system further comprises a rules engine 270 lying between the patient data store 230 and the knowledge base store 260 on one side and the user interface 280 on the other side. The rules engine 270 has the function of combining data from the knowledge base store 260 and the patient data store 230 in response to inputs from the user interface 280 and of providing contextualised advisory information to the user interface 280 for display to the user.

Thus, as the user works through a workflow, each time a user calls up a page the rules engine 270 checks whether the page ID is associated with a particular rule in the monitoring rules table or with a particular subsidiary knowledge base. If the page ID is associated with a rule in the monitoring rules table or a particular subsidiary knowledge base, the advisory information associated with the rule is passed by the rules engine 270 to the user interface 280 for display in accordance with the rule.

For example, when page P002 is called up in workflow 3, the rules engine 270 establishes that the first rule in the table in Fig. 22 might apply, checks from the patient data store 230 whether the advisory information associated with that rule was displayed more than 12 months ago and, if it was, passes the wording "Send reminder to primary care team" to the user interface 280 to display in the Communications section of page P002.

An example is shown in Fig. 7, which shows pages P001 and P002 displayed at the same time, together with the guidelines area 50. In this case, the first rule in the table is not invoked because less than 12 months has expired since the first visit and wording "Send reminder to primary care team" is not displayed in the guidelines area 50. However, the guidelines area 50 is caused to display that the NICE clinical guidelines recommend that on this visit lipid profile labs are ordered, blood pressure and weight are measured, and that a PANSS is completed.

Moreover, additional proprietary guidelines included in the personalised medicine system 1, 100 recommend that, for example, a glucose test is ordered on a more frequent basis than the NICE guidelines recommend (and gives the date of the last result available for a glucose test), and gives the reason that, for example, the patient has a family history of diabetes as well as the last glucose level being high.

In this case, a more complicated rule has been used for the proprietary guidelines than the exemplary rules shown in the monitoring schedule table in Fig. 24. In particular, the proprietary guideline uses a rule that checks the family history of the patient, the date of the last test result for glucose and the glucose level of the last test result to determine whether to recommend performing a glucose test. Thus, based on the page ID, the rules engine 270 extracts the relevant rule(s) and any supporting information that may need to be displayed from the appropriate subsidiary knowledge base KB stored in the knowledge base store 260 and any required information from the patient information store 230 required for the extracted rules. The rules engine 270 then runs through each of the extracted rules, using the retrieved information from the patient data store 230 as necessary, to determine if any advisory information should be displayed in the guidelines area 50 and, if so, what. Depending on the result, the rules engine 270 passes the relevant advisory information from the knowledge base store 260 and the patient data store 230 to the user interface 280 for display on the display device 1802. The user may then click on or otherwise select some of the displayed advisory information to cause the user interface 280 to retrieve (if not received with the initially displayed advisory information) the secondary wording.

In general then, the personalised medicine system 1, 100 searches the knowledge base store 260 for information related to the activity being performed (the stage in the workflow - step, node or page) - in another example, similar to the rule shown in Fig. 23, the clinician wants to treat the patient with an antipsychotic medication.

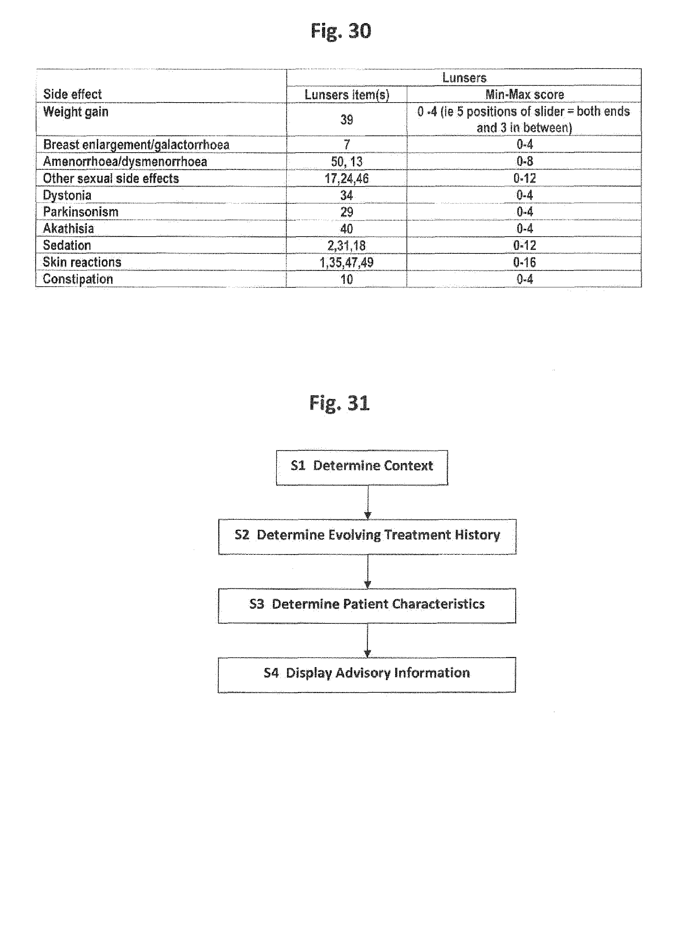

In more general terms, the personalised medicine system 1, 100 examines the context (step S1 in Fig. 31)- in this example, is the working diagnosis first episode psychosis? In this example, NO.

**[0107]** The personalised medicine system 1, 100 then examines the evolving treatment history (step S2 in Fig. 31). In this example, has there been past treatment with 2 or more antipsychotic drugs? YES. Was one of them second generation (ie atypical)? YES. Has there been an adequate trial of the current medication for 4 or more weeks? YES. Has there been a poor response to the current antipsychotic drug? YES. Has clozapine already been trialled? NO.

**[0108]** The personalised medicine system 1, 100 then examines the patient's characteristics (step S3 in Fig. 31). In this example, has there been a history of cardiovascular (CVS) disease? YES.

The personalised medicine system 1, 100 then offers the relevant guidance (step S4 in Fig. 31) - in this example, the guidance is to review diagnosis, evaluate adherence, consider if the current drug has been prescribed at an adequate dose for the correct duration, trial clozapine, do an ECG, discuss the drug with the patient, and record baseline symptom and side effect ratings. This advisory information may all be displayed at the same time, or may be displayed at different relevant times as the workflow progresses. Thus, the recommendation to do an ECG may only be displayed in the guidelines area 50 when the "order investigations" node is reached on the workflow.

It will be appreciated that the information stored and the processes performed may be distributed otherwise than as described above to achieve the same functionality. For example, rather than storing (all of) the advisory information, the knowledge base store 260 may store a link to the appropriate source 240, 242, 244, 246, 248, 250 and retrieve some or all of the required information on demand from the rules engine 270, or the rules engine 270 may retrieve the required information, possibly via the knowledge base store 260.

In addition, the rules engine 270 may receive feedback information from the user interface 280 to adapt the advisory information to be displayed. For example, where the knowledge base store 260 provides advisory information from a plurality of different guidelines (for example, one or more published guidelines and/or a proprietary guideline), the personalised medicine system 1, 100 may enable the user to select the guideline(s) on which advisory information should be based.

It should be noted that the advisory information need not always be displayed in the guidelines area 50. Rather, the advisory information may also be used to populate specific information in one or more pages. For example, page P002 at the bottom of Fig. 7 shows actions due based on rules. It should be noted that in the screen shown in Fig. 7, not only are two pages displayed but the guidelines area 50 also includes further advisory information based on the NICE clinical guidelines and proprietary guidelines.

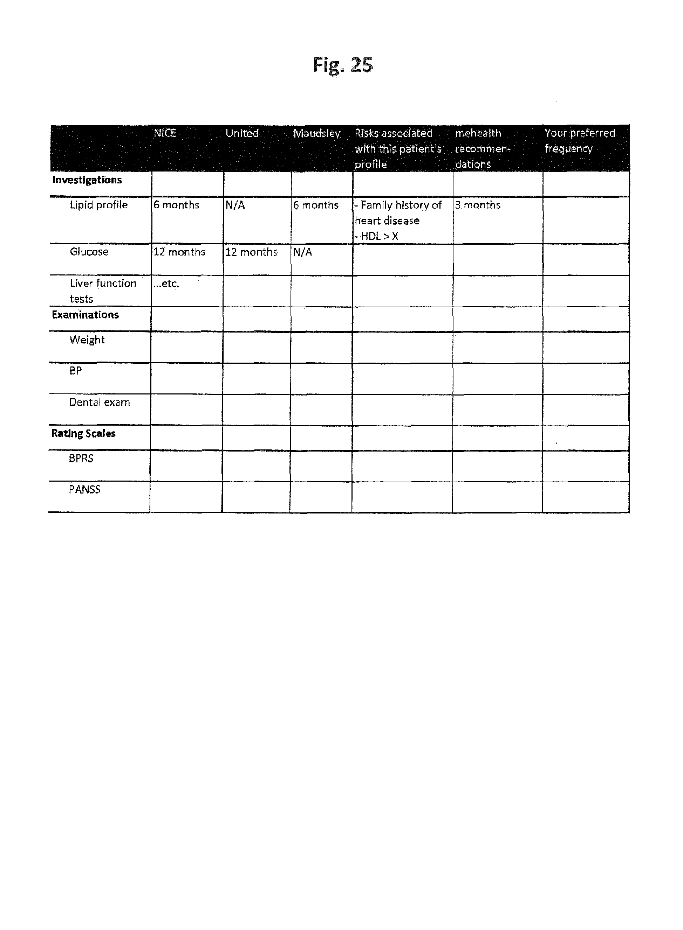

The personalised medicine system 1, 100 may also allow the user to adapt some of the rules, or how some of the rules are operated. For example, the subheading 65 displayed when the main heading 60 highlights the Review Data step in the workflow may include the wording "Open Monitoring Schedule". When this wording is clicked on, the user interface 280 displays a table as illustrated in Fig. 25, which is shown by way of example and is only partially populated. The table shows each of a number of items that should be regularly monitored, such as lab investigations (including lipid profile, glucose, liver function tests); physical examinations (including weight, blood pressure, dental exam); rating scales (including BPRS, PANSS used to monitor symptoms for example) etc. The table further provides a column for each available guideline and shows the frequency of monitoring recommended by each guideline for each item.

In this embodiment, one of the guidelines is a proprietary guideline and the table includes a column showing the risk factors associated with the particular patient's profile that have contributed to the frequency of monitoring recommended for each item by the proprietary guideline based on the rules in the knowledge base store 260 and the data stored in the patient data store 230 for that patient. The user is able to adjust the frequency of monitoring that will be recommended for that patient in the last column. In subsequent operation, the personalised medicine system 1, 100 will provide recommendations for monitoring in the actions due page (P002) based on the proprietary guideline, unless overridden by the user selection, and will provide further advisory monitoring information based on the other guidelines (as selected by the user) and possibly additional information from the proprietary guideline in the guidelines area 50.

Accordingly, the personalised medicine system 1, 100 of the present invention displays context specific advisory information to the user as the user works through a workflow. More particularly, the advisory information displayed is specific to the context of the stage in the workflow (step, node, page) and to the patient data of the patient in consultation. Thus, the personalised medicine system 1, 100 of the present invention provides the clinician with the most appropriate information for that stage, specifically tailored to that patient. Thus, the personalised medicine system 1, 100 drives a higher standard of care than may be expected from a perusal of the guidelines alone.

Moreover, the patient data evolves both as an individual consultation progresses and as the number of visits grows and treatment progresses. The personalised medicine system 1, 100 updates the advisory information displayed as the patient data evolves to ensure the clinician is always presented with the appropriate advisory information in the context of the changed patient data and the stage of the workflow. Because of the way the stages (steps, nodes, pages) in the workflows are ordered, the personalised medicine system 1, 100 can ensure that the appropriate data is gathered before recommendations are made in subsequent stages.

Moreover, as the sources of data 240-250 for the knowledge base store 260 evolve, so the knowledge base store 260 can evolve and be updated. This can occur automatically as the sources 240-250 change. Thus, if the rules lead to display of information from the prescribing data source(s) 246, and the data from the data source changes, this can be reflected in the data shown by the personalised medicine system 1, 100. For example, updates in the prescribing data 246 can be automatically imported into the knowledge base store 260, or the knowledge base store may provide a link to the same place, and the information at the link will change as the source evolves. Naturally, this applies to the other sources of data 240-250.

Moreover, the rules in the knowledge base store 260 may be updated automatically or manually too.

From the foregoing discussion of the general architecture, it will be apparent that each of the patient data store 230, the knowledge base store 260 and the rules engine 270 can be stored on the clinician's computer 1 in one embodiment. Alternatively, in other embodiments one or more of these may be stored on a remote server/computer 130, 140 and the clinician's computer 110 may access them via LAN, WAN and/or the Internet. Such an arrangement might be preferable, for example, where the clinician uses a tablet computer 110 during consultation to maximise interaction with the patient. In this case, the clinician's computer 110 embodying one aspect of the invention may store only the user interface 280 component as an application or widget and access the other components of the system by Wi-Fi.

In another embodiment, the user interface 280 and the rules engine 270 are stored on the clinician computer 110, the patient data store 230 is stored on a server 130 for a clinic, and the knowledge base store 260 is stored on a separate server 140. Multiple clinicians' computers 110, for example within a single hospital or health provider, may access patient data held by the patient data store 230 on the server 130. In addition, clinician's computers 110 for multiple clinics or health providers may access the knowledge base store 260 held on the server 140, which is maintained and updated by a third party.

It will be apparent that various other distributions of physical and software components of the invention are possible, and each is within the scope of the present invention.

*Side Effect Risk and Drug Mapping*

As described above, the personalised medicine system 1, 100 calculates, for a plurality of potential side effects, a risk that the patient will experience the side effect relative to the general population and displays the side effects in ranked order on page P018, as illustrated in Fig. 18. The personalised medicine system 1, 100 then displays the likely suitability for treatment of a patient of each of a list of drugs based at least in part on the calculated side effect risks. The manner in which the relative side effect risks and ranked order are determined, as well as the mapping of the side effect risks to the list of drugs will now be discussed in more detail.

As previously discussed, as the clinician works through the workflow during a consultation with a patient, he optionally carries out or populates patient information, for example side effect rating scales in page P010 (Fig. 13) and physical exams in page P011 (Fig. 17) and investigations in page P022. In addition, the clinician may change the side effect ratings by using the side effect sliders 80, illustrated in Fig. 15. The patient may also have access to an interface which allows him to record the severity of side effects and the distress which they cause.

Preferably, the personalised medicine system 1, 100 also offers the ability to include side effects previously experienced by the patient. These can be recorded by the clinician during a consultation based on the patient's previous clinical history before the personalised medicine system 1, 100 of the present invention was used for the patient, for example using workflow 2. In addition, the previously experienced side effects can be data recorded in previous consultations since the personalised medicine system 1, 100 was used for the patient. This previously recorded data can be the position of the sliders during a previous consultation or, if the patient stops treatment using a medication and the clinician records why. The data on the side effects previously experienced by the patient is used to position the vertical line on the slider 80 in Fig. 15.

This patient data is then used to calculate the relative risks and populate page P018 in Fig. 18. In particular, the present invention recognises that there are a number of risk factors (or moderators) that can affect each side effect, and uses these to assess an individual patient's risks of experiencing each side effect.

More specifically, the personalised medicine system 1, 100 stores, for example in the knowledge base 260, a table of influences used to calculate relative risks. The table of influences is multidimensional and includes a number of different layers (or conceptually, comprises a number of related tables).

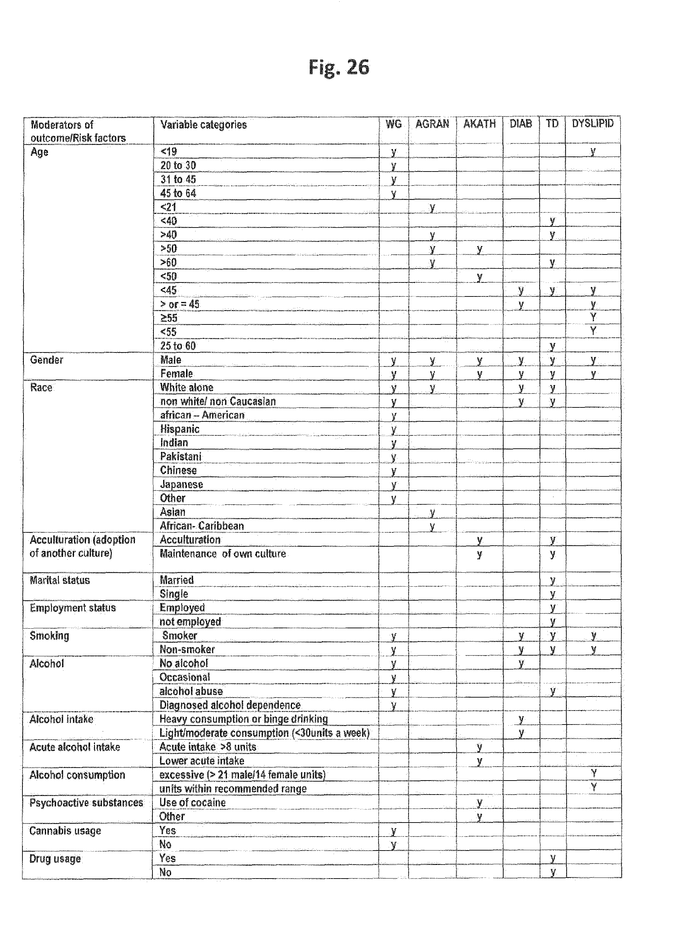

In a first layer, the table lists all of the different moderators that can affect the risk of experiencing a side effect together with (sub-) categories for each moderator and an indication of which categories affect which side effects. An exemplary extract of this layer of the table is shown in Fig. 26. The first column shows the moderators, the second shows each of the categories for each moderator and the subsequent columns show various side effects - in this case, weight gain (WG), agranulocytosis (AGRAN), akathisia (AKATH), diabetes (DIAB), tardive dyskinesia (TD), and dyslipidemia (DYSLIPID).

Each of the moderators relates to patient-specific information and it can be determined whether the patient falls into one of the categories of a moderator by extracting the relevant patient information stored in the patient data store 230. It can be seen that the categories within a moderator can overlap. Thus, in the example in Fig. 26, the age moderator includes the overlapping categories 31-45, <40 and 25-60. This is because the different side effects are affected differently by the different categories.

In the exemplary table in Fig. 26, only six side effects are shown. However, it is preferred that a more exhaustive list of side effects is used (even more exhaustive than the example shown in Fig. 15). Similarly, the table in Fig. 26 shows only a limited number of moderators, but in practice many more moderators will be used including not only moderators relating to personal information such as those shown in Fig. 26 but also family history moderators; medical physical history moderators (eg cardiovascular disease, obesity, liver disease); psychiatric history moderators; baseline investigation moderators (eg baseline lipid profile, baseline white blood count pre-clozapine); current investigation moderators; physical examination moderators (eg body mass index); current clinical moderators (eg current diagnosis, schizophrenia sub-type); past clinical moderators (eg time since first antipsychotic treatment); current medication moderators (eg dose); and so on.

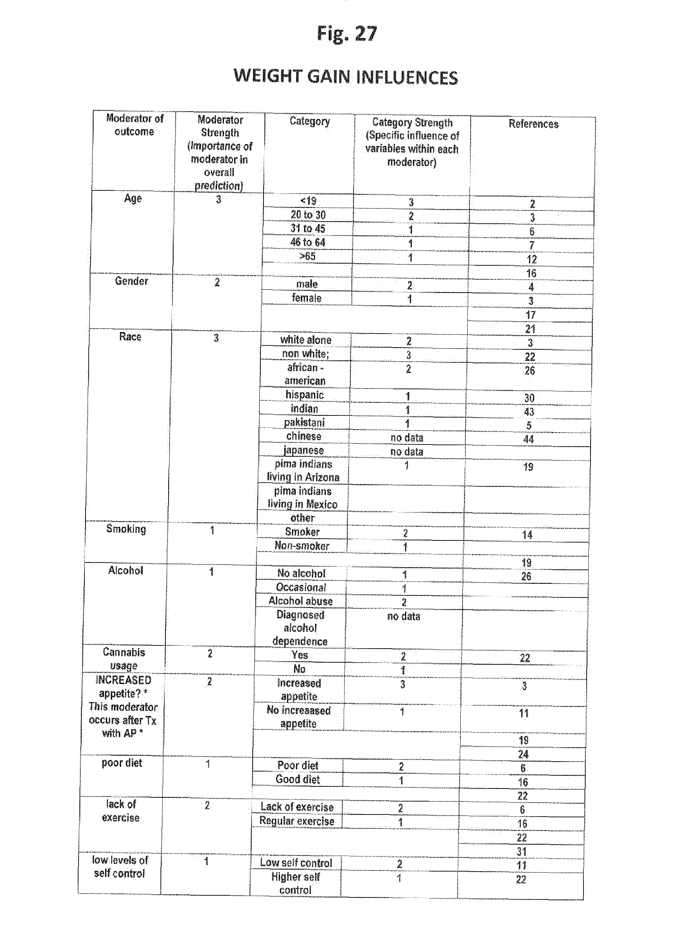

A separate layer of the table is provided for each side effect. Fig. 27 shows an extract of a layer provided for the side effect of weight gain. In the table, each moderator is assigned a moderator strength within a predetermined range (1-3 in the example) in accordance with the effect the moderator has on the likelihood of the patient experiencing the side effect relative to the other moderators. In addition, each category is assigned with a category strength within a predetermined range (1-3 in the example) in accordance with the effect the category has on the likelihood of the patient experiencing the side effect relative to the other categories for that moderator.

Thus, in the table layer shown in Fig. 27, it can be seen that the moderator of age has a stronger effect that the moderator of gender on the risk of a patient experiencing the side effect of weight gain. Moreover, patients under 19 years old will be more likely to experience weight gain than a patient 20 to 30 years old, who in turn will be more likely to experience weight gain than older patients. Moreover, male patients are more likely to experience weight gain than female patients.

In order to determine a risk indicator for the patient experiencing a particular side effect, the personalised medicine system 1, 100 interrogates the table of influences and determines which factors moderate the risk of experiencing the side effect, and which categories of each relevant moderator the patient falls into. The personalised medicine system 1, 100 then combines the moderator strengths and category strengths to determine an overall risk indicator for that side effect.

Any suitable way of combining the moderator and category strengths may be used. Preferably, the moderator strength ($M_x$) of each relevant moderator is multiplied by the category strength ($C_x$) of the category in which the patient falls, and the results of the multiplications are summed to reach a total risk value V:

$$V = (M_1C_1 + M_2C_2 + M_3C_3 + ... + M_nC_n).$$

For example, referring to Fig. 27, a male under 19 years old would be 2.6 (13/5) times as likely to experience weight gain than a female over 65 years old, all other factors being equal. (3x3 + 2x2 = 13; 3x1 + 2x1 = 5).

Where n is the number of moderators for which information is available to classify the patient, Mmax is the maximum value for the moderator strength and Cmax is the maximum value for the category strength, a risk indicator I for a side effect can be calculated as:

$$I = V/(n * Mmax * Cmax).$$

In the foregoing example, if only age and gender were known, the final risk indicator In for weight gain for the male under 19 years old would be 0.72 (13/(2*3*3)) for the female over 65 years old it would be 0.28 (5/(2*3*3)). Thus, by dividing the risk value V in this way, it is possible to obtain a value between 0 and 1 for each risk indicator.

For any one side effect, the category strengths need to be normalised across a predetermined scale. For example, in Fig. 27, all category strengths are between 1 and 3. This ensures that the values assigned to moderator strength properly reflect the strength of the moderator relative to the other moderators for the side effect, and the combined moderator and category strength results are not unduly influenced by the category strengths.

In addition, in order that risk indicators for different side effects can be usefully compared, if the range of category strengths differs between side effects, it is normalised for all side effects, for example to a scale of 1 to 9. This can be done in a number of ways but in the present embodiment normalisation is achieved by mapping category strengths for a side effect to a normalised scale in a predetermined way. Where the range of category strengths is from 1 to 9, no mapping is required. Where the range of category strengths is 1 or 2, 1 is mapped to 1 on the normalised scale and 2 is mapped to 9. Where the range of category strengths is 1 to 3, 1 is mapped to 1, 2 is mapped to 5 and 3 is mapped to 9 on the normalised scale. Where the range of category strengths is 1 to 4, 1 is mapped to 1, 2 is mapped to 3, 3 is mapped to 6 and 4 is mapped to 9. Where the range of category strengths is 1 to 5, 1 is mapped to 1, 2 is mapped to 3, 3 is mapped to 5 and 4 is mapped to 7 and 5 is mapped to 9, and so on. Moreover, the moderator strengths are selected to allow useful comparison between the risk indicators determined for each side effect.

It will be apparent that risk indicators can be determined for each side effect using different modes of combining moderator and category strengths or using different ways of ranking moderators/categories. For example, rather than multiplying moderator and category strengths, they can be added and the results for all moderators can be added. However, some normalisation is preferred so that risk indicators for the different side effects can be usefully compared with one another.

Once the personalised medicine system 1, 100 has calculated a risk indicator for each of the side effects, they are compared and the side effects are displayed in page P018 in order of, or with some indication of, the respective risk indicators. Thus, in Fig. 18 the relative risk of the patient experiencing dystonia is highest and so dystonia is displayed at the top in the largest font, and the relative risk of diabetes is lowest so diabetes is displayed at the bottom in the smallest font.

It should be noted that in the present embodiment, the risk indicator is an indication of the selected patient's risk relative to other patients. For example, if the patient is a 19 year old African American male with mid-range BMI, he is at a moderately higher risk than the general population of experiencing weight gain. However, he is at a lower risk than the general population of experiencing akathisia, and a higher risk than the general population of experiencing dystonia. However, although dystonia may be listed first and weight gain fourth, this does not mean he is more likely to get dystonia than weight gain. This is because weight gain is a more common side effect than dystonia.

If desired, each side effect can also be provided with a side effect strength S, which is multiplied by the risk indicator for the side effect to give a final side effect likelihood L (or risk indicator that has been modified):

$$L = S * I$$

In some embodiments, the user may be able switch between showing the side effects in order of L or I, or these may be shown concurrently, although this is not necessarily preferred.

The table in Fig. 27 also includes a column including references. When the user clicks on the "i" icon next to a side effect on page P018, he is taken to the references referred to in the table for each moderator and/or category and stored in the knowledge base store 260. Thus, if the user clicks for information relating to weight gain and the patient is under 19 years old, the user will be displayed the text of references 2 and 16 stored in the knowledge base store 260 or accessed from there but stored in peer consensus source 244, for example. The table may also include a table for comments to be displayed in the event the patient falls within a particular category used in the determination of the risk indicator.

In general existing practice, it is not possible for clinicians to take account of the large numbers of different factors that affect the likelihood of a patient undergoing treatment, particularly during a consultation, and clinicians rely on their own experience in assessing the likelihood of side effects when prescribing medications. However, the present invention provides the user with a side effect risk profile for the selected patient relative to the general patient population, which is a powerful tool in guiding the clinician during a clinical consultation. Moreover, the relative risk profile can take account of a vast array of sources of primary data to which the clinician may not have had access and will not be aware, and which may be counterintuitive to his own experience. Further, the relative risk profile is provided at an appropriate step in the consultation workflow, ensuring that it will be considered by the clinician at the appropriate time. Thus, the present invention significantly improves patient care and outcomes. The skilled addressee will appreciate that the side effect relative risk profile can be shown in any number of different ways, whilst still providing the foregoing advantages and benefits.

As noted above, the user may select side effects to be avoided. Depending on the working diagnosis selected in page P015 and, optionally, the side effect relative risks calculated by the personalised medicine system 1, 100 and the patient's own preferences the side effect(s) selected to be avoided in page P018 and other patient data, the personalised medicine system 1, 100 then displays in a particular order a list of suitable AP drugs for treatment of the patient (see Fig. 19) in page P019.

The drugs used to populate the list in page P019 will be selected based on rules and information stored in the knowledge base store 260, as described above. For example, the list of drugs may correspond to the 10 most popularly or commonly described drugs taken orally and the five most commonly prescribed drugs taken by injection in the knowledge base for patients having the same diagnosis and similar characteristics.

Page P019 further includes an indicator (shown on the left hand side in Fig. 19) of the relative risks of the displayed drugs to the particular patient. In particular, in the example in Fig. 19, a single exclamation mark (!) is placed against drugs 6-8 in the first list and two exclamation marks (!!) are placed against drugs 9 and 10. Similarly, a single exclamation mark (!) is placed against drug 4 in the second list and two exclamation marks (!!) are placed against drug 5.

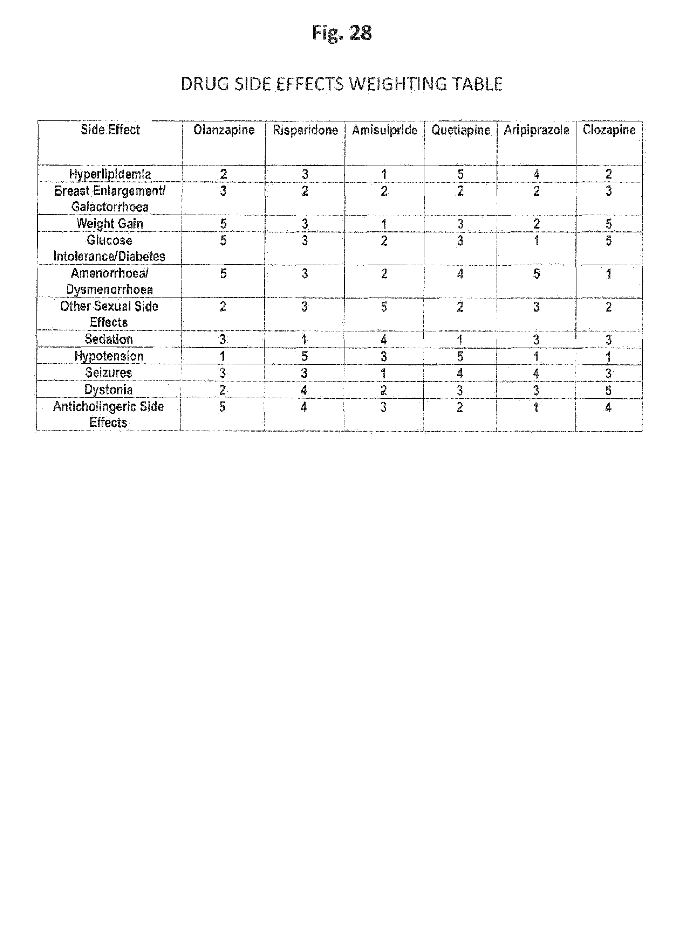

The determination of the order in which the drugs are displayed and of whether to place any exclamation marks against a drug may be carried out as follows. The knowledge base store 260 further holds a drug side effects weighting table, an exemplary extract of which is shown in Fig. 28. For each drug, a weighting is given for each of the possible side effects, reflecting the likelihood that the drug will cause the side effect. The weighting is given as a number within a predetermined range, in this example 1-5. Generally, the weightings reflect a likelihood of the drug causing patients to experience the side effect relative to the other drugs. However, in some embodiments the weighting may reflect an absolute likelihood of an average patient experiencing the side effect as a result of taking the drug.

In the present invention, the rules engine 270 retrieves the drug side effect weightings table from the knowledge base store 260. Then, for each drug in the table, the rules engine 270 multiplies the risk indicator I for each side effect previously calculated for the patient and multiplies it by the weighting W for the corresponding side effect. The results are then summed to obtain an overall drug weighting score Dw for the drug:

$$Dw = I_1W_1 + I_2W_2 + ... + I_nW_n$$

Thus, an overall drug weighting score Dw specific to the patient can be determined for each drug, providing a clinician with an overview of how likely any particular drug is likely to cause the specific patient in consultation to experience side effects. This overall drug weighting score Dw can feed into the determination of whether a drug is suitable for a particular patient.

Although not preferred, it would be possible to use the overall drug weighting score Dw to determine whether to exclude particular drugs from being displayed in the list discussed above. For example, if Dw for a particular drug were to be larger than a predetermined threshold, that drug could be removed from the list. Instead, or as well, the overall drug weighting score Dw could be used to order the drugs in the list.

The 10 most prescribed drugs (for example for the hospital, clinic, region, healthcare provider, insurance provider, or country etc) for patients having the same diagnosis will be displayed in order of their respective overall drug weighting scores Dw. The overall drug weighting scores Dw may be compared to different thresholds to determine whether to display one or two exclamation marks next to them. Thus, the number of exclamation marks displayed next to each drug will vary between patients, although the drugs included in the list will not.

Only the most prescribed drugs are shown to avoid the clinician needing to look through a long list of drugs he is unlikely to prescribe. Naturally any number other than 10 may be shown. Moreover, the user may opt to view the full list of drugs, which will each be presented with no, one or two exclamation marks next to them in the same way.

In a preferred embodiment, in addition or instead, the results of each individual multiplication InWn are compared with one or more thresholds. If any one of them falls above a lower threshold, then the drug may be displayed with one exclamation mark and if any one of them falls above the upper threshold, the drug may be displayed with two exclamation marks.

Thus, as explained above, a single exclamation mark may be used to indicate that the drug should be used with some caution, as the patient's profile suggests that he may have an increased risk of one or more adverse side effects. Two exclamation marks may be used to indicate that the drug should be used with extreme caution, as the patient's profile suggests that he is likely to have an increased risk of one or more adverse side effects. Naturally, the number of thresholds and the number of exclamation marks (or other indicators) may vary.

Once the list of most prescribed drugs has been retrieved, it may be displayed in the order of drugs for which no value of InWn is above the lower threshold, in the sub-order of overall drug weighting score Dw; drugs for which one or more values of InWn is above the lower threshold, in the sub-order of the number of values InWn over the lower threshold and the sub-sub-order of overall drug weighting score Dw; and drugs for which one or more values of InWn is above the upper threshold, in the sub-order of the number of values InWn over the upper threshold, the sub-sub-order of the number of values InWn over the lower threshold and the sub-sub-sub-order of overall drug weighting score Dw. The user may also be able to command the user interface 280 to display all available drugs rather than just the top 10 or 5, and these may be displayed with exclamation marks in the same way. Optionally, the list of all the drugs may be re-ordered as discussed above.

As noted above, a clinician may select a side effect which the patient wishes to avoid. This may be fed back to the rules engine 270 to adjust the calculation described above. For example, the weightings W in the drug weightings table for the selected side effect and/or the patient's relative risk indicator may be multiplied by a predetermined coefficient to increase its significance in the value of InWn and Dw. Alternatively, the threshold(s) for the side effect may be lowered.

Accordingly, the drug table provides a wealth of information tailored to the specific patient to allow the clinician and the patient to discuss suitable AP drug treatment options and select an AP drug for treatment. In general existing practice the risk specific to a particular patient of experiencing side effects due to a drug has not previously been considered during patient consultations. By providing patient relative risk indicators and mapping them to drugs, the present invention allows clinicians to avoid a large amount of trial and error in finding the most suitable drugs to treat patients, thereby providing significantly improved outcomes in less time and saving healthcare providers significant sums of money.

*Timeline*

A further innovative aspect of the present invention is the provision of a timeline. In more detail, a timeline 400 is displayed before commencing the workflow and during the display of the pages as the workflow proceeds.

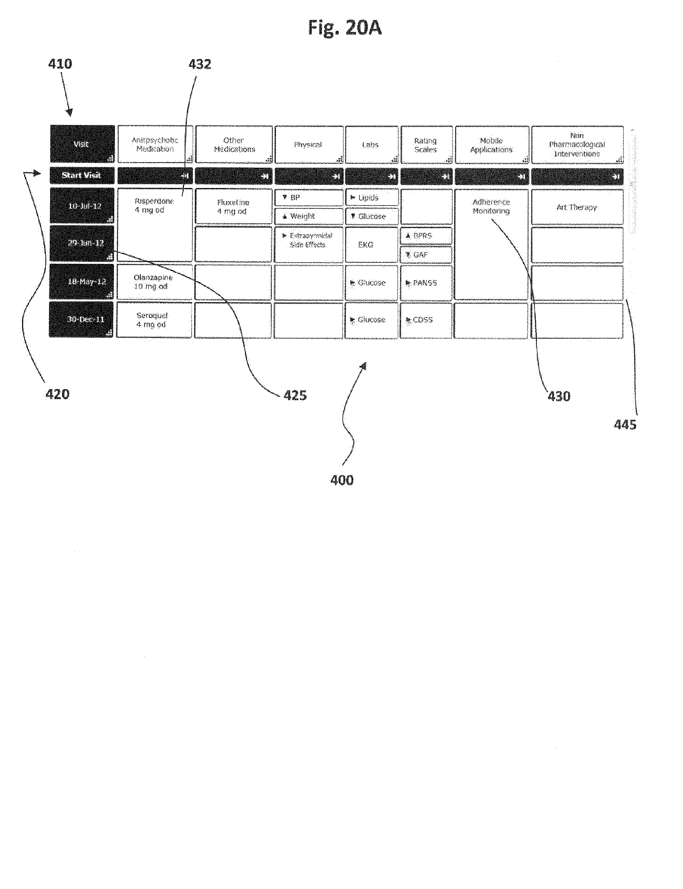

An exemplary timeline 400 is shown in Fig. 20A. This timeline is shown when the user first logs on to the personalised medicine system 1, 100 and selects a patient for consultation. The timeline comprises a header row 410 along a horizontal axis, together with visit rows 420, 425 arranged below the header row 410 along a vertical axis and extending along the horizontal axis.

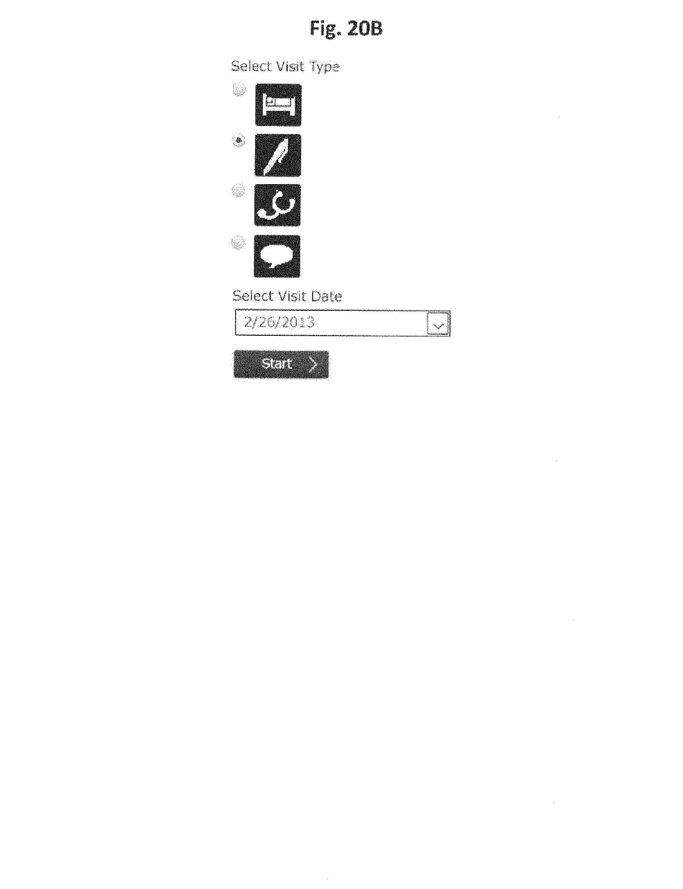

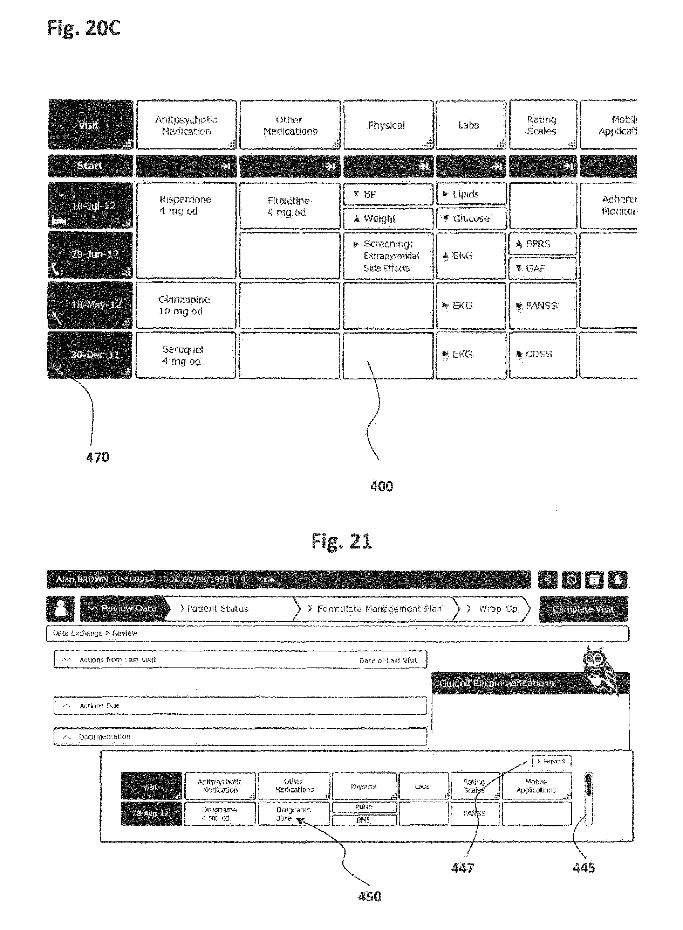

The header row 410 includes headings for each of a plurality of different categories of patient data. In the example in Fig. 20, these categories are antipsychotic medication; other medications; physical (height, weight, blood pressure etc); labs (lipids test, glucose test etc); rating scales (BPRS, PANSS etc); mobile applications (installed on the patient's smartphone or tablet for remote monitoring); and non-pharmacological interventions (CBT, gym, art etc).

Each of the visit rows 420, 425 shows the patient data recorded for the patient for a particular visit. Thus, in the first column of a visit row 425 the date of the consultation is displayed. In subsequent columns of the visit row, the patient data for that visit is displayed. Accordingly, taking the example shown in Fig. 20, it can immediately be ascertained that the patient visited the clinic on 30 December 2011, 18 May 2012, 29 June 2012, and 10 July 2012.

In the visit of 30 December 2011, a CDSS rating scale assessment was performed, a glucose test was ordered and quetiapine was prescribed at 4 mg once per day. The arrows 440 indicate that the results of the glucose test and the CDSS were unchanged (or not significantly changed) from previous results.

During the visit of 18 May 2012, a PANSS assessment was performed, another glucose test was ordered and the antipsychotic prescription was changed to olanzapine at 10 mg once per day. In addition, adherence monitoring was commenced using the patient's smartphone.

During the visit of 29 June 2012, BPRS and GAF assessments were performed, an ECG (EKG) test was ordered, and an assessment of extrapyramidal side effects was carried out. In addition, the antipsychotic prescription was changed again to risperidone at 4 mg once per day. In addition, as indicated by the merged cell 430, adherence monitoring was continued.

Finally, during the visit of 10 July 2012, lipids and glucose labs were ordered and blood pressure and weight were measured. Since last being measured, lipids were constant and glucose levels were reduced, weight having increased and blood pressure having fallen. The antipsychotic prescription risperidone at 4 mg once per day was continued, as indicated by the merged cell 432. In addition, the non-AP medication fluoxetine was prescribed at 4 mg once per day. Adherence monitoring was continued and art therapy was commenced.

Accordingly, at the beginning of a consultation the clinician can quickly assimilate the treatment history of the patient, with details being shown in such a way that changes/continuations in symptoms/treatments/other factors can be seen at a glance. Preferably, data gathered before use of the personalised medicine system 1, 100 and subsequently entered is clearly separated from other data in the timeline, for example by using a different colour, shading or font, or a combination of these.

Advantageously, the timeline is interactive. If the user clicks on one of the headings in the heading row 410, the personalised medicine system 1, 100 is caused to display a series of graphs in a pop-up charting window, illustrating graphically how the different factors recorded in the respective category have changed over time. For example, if the user clicks on the Physical heading, he will be shown graphs of changes in each of the patient's measured physical characteristics, such as pulse rate, blood pressure, temperature, weight, BMI, and so on.

Alternatively, if the user clicks on a cell in the timeline, he is taken to the page in the workflow where the information was entered. For example, clicking on Pulse will take the user to the physical exam page P011 for the visit in which Pulse is located. Effectively then, the personalised medicine system 1, 100 is able to recreate previous workflows and allows the user to navigate through them as with a current workflow. The user may be enabled to edit previous visits, for example to correct previously input information or to add new information. The personalised medicine system 1, 100 informs the user when they are editing a previous visit. Preferably, all input fields within a completed visit are initially disabled, but the user is given the ability to edit a page via a banner at the top of a page that appears when the user first attempts to edit the page.

If the user mouses over a cell in the timeline, a pop-up window may be displayed to see further details of the patient characteristics in the cell -for example, the patient's weight at that visit.

As previously discussed, some cells within the timeline are merged. In more detail, cells under headings such as "Antipsychotic Medication", "Other Medication" and "Mobile Applications" are merged across visits where there is continuity between the visits. However, separate cells are started where there has been a change.

Where there have been a large number of visits, the timeline is provided with a scroll bar 445 so the user can scroll between earlier and later visits.

It is preferred that the personalised medicine system 1, 100 allows a distinction to be made between the different times data may be added to the system. To differentiate between the types of data in the personalised medicine system 1, 100 in a preferred embodiment, four visit types are provided: new visit (of the patient to the clinic); admission (an event where a patient is admitted into a clinical setting, for example as an inpatient at a hospital); encounter (an encounter with the patient outside of a scheduled visit, for example by phone or e-mail; and data entry (any data collected between visits to be included as part of the patient record, such as the results of lab tests). Naturally, these are not limiting.

The first row 420 in the timeline (under the heading row 410) is the Start row. If the user clicks on the Start cell (shown as Start Visit in Fig. 20A and Start in Fig. 20C), the personalised medicine system 1, 100 displays the information shown in Fig. 20B and gives the user the option of selecting the appropriate visit type. If the user inputs that the visit is a new visit by the patient to the clinic, the current date will populate the start cell. Otherwise, the user can choose the date to associate with the admission-, encounter- or data entry-type visit. An icon 470 is placed in the visit identifier in the first column, as shown in Fig. 20C, so that the user can distinguish between different types of visit when viewing the timeline 400 later. The personalised medical system 1, 100 this will begin the appropriate workflow and start the display of the respective pages as the workflow progresses.

As shown in Fig. 21, in a preferred embodiment a minimised timeline 450 is permanently shown at the bottom of the display device 1802 as the user progresses through the workflow. The minimised timeline includes the header row 410 and, in the first instance, the current visit row 420 created by clicking in the start cell. If the user has entered the workflow by clicking on a cell from a previous visit, the row corresponding to the previous visit will initially be displayed in the minimised timeline. (The header row in the embodiment shown in Fig. 21 is different from that shown in Fig. 20 simply because two different embodiments of the personalised medicine system 1, 100 are shown. However, if the figures were to relate to the same embodiment, the same headers would be shown in the header row 410 of the two figures.)

As the user progresses through the workflow and inputs patient data (whether in the current visit or a previous visit), this is immediately reflected in the timeline. In other words, the timeline is populated directly patient information is entered/changed. Again, the user can click on cells in the minimized timeline to navigate to the relevant page in the workflow to enter the relevant patient information.

Accordingly, in addition to being able to navigate through the workflow sequentially as described above by clicking forward/next and back/previous buttons or by clicking on the workflow steps and nodes in the display header 60 and sub-header 65, the full timeline 400 and the minimised timeline 450 provide the user with a further way of navigating through the workflow.

As shown in Fig. 21, the minimised timeline 450 is provided with a scroll bar 445 so he can scroll through previous visits even as he is going through the current workflow. The minimised timeline 450 is also provided with a maximise button 447 to allow the user to view the full timeline 450 overlaid on the page display. When the timeline has been maximised in this way, the button 447 operates as a minimise button to allow the user to return to the minimised timeline 450.

As such, the timeline is a powerful tool giving the user quick access to a summary of a patient's progress since initial interaction with the personalised medicine system 1, 100, and even beforehand if the relevant data is correctly input. The timeline is available from all major screens in the personalised medicine system 1, 100. This allows the user to quickly scan through a summary of past visits as well as the data already entered during the current visit even as the user is entering more detailed information using the various pages in the workflow. Moreover, the user can see trends in the data from the timeline due to the arrow indicators 440 and quickly gain access to more detailed information by mousing over a populated cell or by drilling down to the detailed information by clicking on the cell or a heading in the header row 410. As such, the timeline provides many unique advantages in fully informing a clinician's decisions as a consultation progresses.

*Patient Characteristic Scale Display - Sliders*

As previously discussed, graphical scales can be automatically populated to display various patient characteristics, such as symptoms (or categories of symptoms) and side effects (or categories of side effects). More particularly, symptoms and side effects experienced by the patient can be assessed using rating scales, with the results being displayed in a graphical linear scale in pages P009 and P012.

More particularly, when the clinician completes one or more symptom or side effect rating scales, the personalised medicine system 1, 100 calculates a value for each of a plurality of predetermined symptoms or side effects for the patient and displays the results of the calculations on a graphical scale, which may be termed sliders 70, 80. In addition to, or instead of, populating the sliders 70, 80 based on the clinician's data input to one or more rating scales, the user is able to enter a value on the scale based on his own impressions or additional information gained during the visit. Thus, each slider 70, 80 includes:

- a first indicator 72, 82 positioned on the scale to represent a normalised value calculated by the personalised medicine system 1, 100 based on completion of one or more rating scales;
- a second indicator 74, 84 positioned manually by the clinician (by which means the clinician enters the value based on his own impressions/additional information);
- optionally a fill 75, 85 along the graphical scale from the lowest value to the second indicator 74, 84; and
- a third indicator 76, 86 representing the most recently set previous value, set during a previous visit (which is positioned in the same place as the second indicator 74, 84 in the previous visit or, if the clinician did not input this, the first indicator 72, 82 in the previous visit).

In the present embodiment, the user enters a value manually by clicking on the selected slider and dragging an icon along the scale to the appropriate position. Alternatively the user may be able to enter a value in box (preferably from 0-10 or 0-100) and this will position the manually input slider on the graphical scale. Other input means are also possible. Moreover, it is not necessary for the graphical scale to be linear and various-shaped scales (eg circles) are also possible. Moreover, it is not necessary for the sliders to be horizontally aligned and any suitable icon or representation can be used, including for the first to third indicators discussed above.

If a value has already been calculated by the personalised medicine system 1, 100 or a previous value has been stored, this will be displayed on the slider and the user will be able to use this information together with his experience to position the manually input slider.

In addition, in some embodiments the patient is provided with a means to enter a value on each of the scales. In this case, the workflow may include a screen intended to be shown to a patient, allowing the patient to enter values in the same way as the user (clinician). Alternatively, it is preferred that the patient is able to enter values using the patient device 120, again by sliding an indicator along the scale, or in any other suitable way.

Where a patient input is provided, it is preferred that the patient is shown a different slider for each characteristic (symptom, side effect) with a fourth indicator for the last value he entered (similar to the third indicator discussed above) and a fifth indicator positioned manually by the patient (similar to the second indicator discussed above). That way, the patient is able to compare how he is currently feeling to how he was feeling last time he made the assessment and to move the fifth indicator accordingly. The person skilled in the art will appreciate that although it is preferred that only the fourth and fifth indicators are displayed to the patient, any one or more of the first to third indicators may also be displayed to him.

The fourth and fifth indicators may be displayed to the clinician on the same sliders 70, 80 discussed above, or separate patient-input sliders may be displayed to the clinician. In that case, each patient-input slider may be displayed may be displayed in the immediate vicinity of the corresponding clinician-input slider 70, 80 so the clinician can compare them with ease.

The manner in which the personalised medicine system 1, 100 populates values to determine the positions of the first icons on the sliders 70, 80 will now be discussed. This aspect of the present invention is predicated on the recognition that the various different scales seek to measure various overlapping symptoms/side effects in similar but different ways. For example, the concept of positive symptoms and negative symptoms is well-recognised in psychiatric medicine. However, these are measured in different ways by the BPRS and PANSS rating methodologies. More specifically, as discussed above, PANSS ratings use a sub-scale comprising seven items testing for positive symptoms, giving a minimum score of 7 and a maximum score of 49 assuming all seven items are completed. By contrast, BPRS uses four items to test for positive symptoms (#4, conceptual disorganisation; #11, suspiciousness; #12, hallucinatory behaviour; and #15, unusual thought content). As with PANSS, each item is ranked from 1 to 7. Thus, the minimum score for positive symptoms from BPRS is 4 and the maximum score is 28, again assuming all four items are completed. There are similar overlaps between other scales.

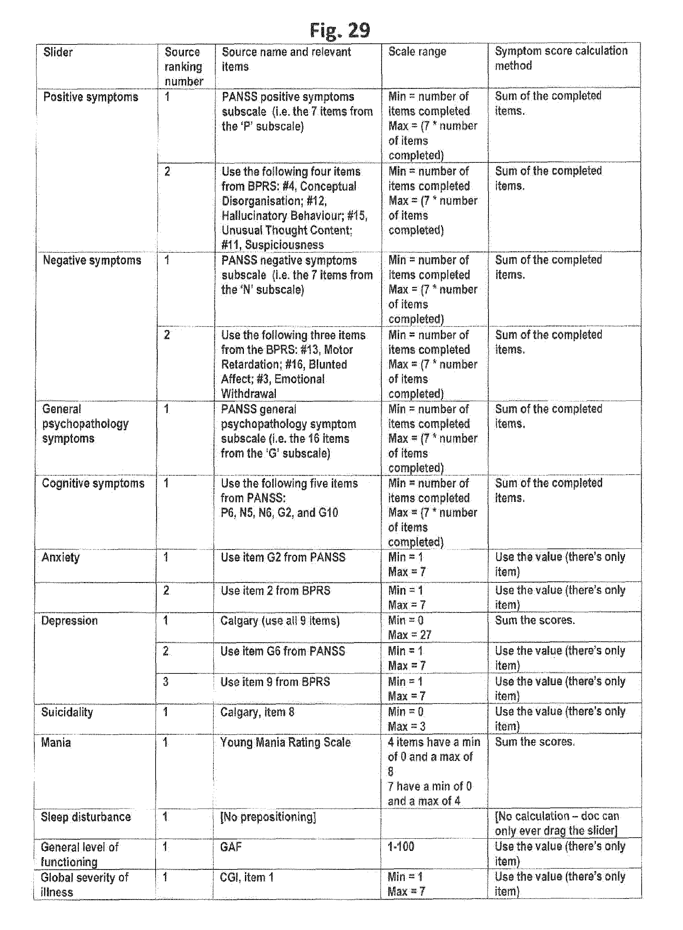

In determining values to populate the symptom sliders, the personalised medicine system 1, 100 interrogates a symptom sliders determination table stored in the knowledge base store 260, an exemplary extract of which is shown in Fig. 29. The table lists each rating scale that can be used to assess for each symptom (or category of symptoms), and which items from each listed scale are pertinent to the assessment of the respective symptom (or category of symptoms). Where two or more scales can be used, they are giving a ranking. In addition, for each source the scale range (minimum to maximum possible scores) is determined. For example, for the positive symptom 'P' scale of the PANSS, the minimum score is (number of items completed x 1) and the maximum score is (number of items completed x 7). The table also includes a calculation method for determining the score for the symptom (or category of symptoms) using the respective scale. For example, if three items of the 7 item 'P' scale are scored during an incomplete PANSS assessment, the values scored for each of the three items are added together. The minimum possible score will be 3 and the maximum possible score will be 21.

To populate the first indicator 72 on the symptom severity sliders 70, the personalised medicine system 1, 100 consults the symptom sliders determination table and extracts the relevant values entered by the clinician when completing the rating scales on pages P008 for each of the symptoms. The personalised medicine system 1, 100 then determines the symptom score for each symptom for which a relevant rating scale has been completed or partially completed.

The determined symptom scores are then normalised for display to a standard range used for all symptoms, with the symptom scores being set to a value on the normalised scale between the minimum and maximum possible scores. In the present embodiment, the normalised scale is a percentage scale. Thus, taking the above example of completing three items of the 'P' scale so the minimum possible score is 3 and the maximum possible score is 21, a score of 3 will be normalised to 0; a score of 21 will be normalised to 1, 100; and a score of 12 will be normalised to 50. Naturally other normalised scales can also be used. However, when the user mouses over one of the indicators or the end points of the scale, the actual value at that point may be displayed.

If the clinician has completed two or more rating scales that relate to a symptom, the personalised medicine system 1, 100 checks to see which has the lowest ranking number (ie is the most important) and discards the results of the other scales from the determination. Generally, ratings scales including a larger number of items relating to the symptom (or category of symptoms) are accorded a lower ranking number (greater importance).

As noted above, the personalised medicine system 1, 100 of the present invention will populate a slider based on an incomplete rating scale. However, if the user mouses over (causes the cursor to hover over) the slider, a hint pops up to inform the user that not all items have been completed. In addition, where two or more rating scales are relevant and at least one has not been fully completed, the personalised medicine system 1, 100 may optionally be configured to accord the lowest ranking number (the most importance) to the rating scale for which the largest number of items relevant to the symptom (or category of symptoms) has been assessed. The user may also be enabled to select which rating scales are used to populate the symptom severity sliders.

The side effect sliders 80 may be populated in a similar way, based on the data entered by the user when completing the rating scales given on page P010. Fig. 30 shows how the LUNSERS rating scale can be used to populate various different side effect sliders. Other ratings scales (eg AIMS and ACS) can also be used to populate these sliders and the interaction between ratings scales is similar to that described above in respect of symptoms.

The personalised medicine system 1, 100 can also populate the side effects sliders taking into account physical patient information entered by the clinician on page P011 or elsewhere. For example, in preference to LUNSERS item 39, the personalised medicine system 1, 100 may use a change in weight or BMI between visits to populate the weight gain side effects slider. In this case, the change may be manipulated to reflect the amount of time between measurements, so that an increase of 5 kg over 6 months is shown as a lower value on the side effects slider than an increase of 5 kg over 2 months. If changes in physical data are used to populate the side effects sliders, they may be discarded if the time between measurements is too large. Although sliders have been discussed above and illustrated in respect of specific rating scales, physical measurements, symptoms and side effects, the skilled addressee will recognise that these are not exhaustive and many other scales measurements, symptoms and side effects could be included, both within the specialisation of mental illness, and for other specialisations to which the present invention can be applied. Moreover, the provision of sliders is not limited to (categories of) symptoms and side effects and can also be applied to other patient characteristics.

Accordingly, the present invention has recognised that while rating scales are a useful tool in assessing patient characteristics such as symptoms and side effects, due to their often complicated structure it can be difficult to extract meaning from their completion. Moreover, the plethora of overlapping scales, each with its own intrinsic value, can further obfuscate a clinician's full understanding. Moreover, the completion of such rating scales can often be very time consuming, so it is difficult for clinicians to complete them on a regular basis, and clinicians therefore often see such rating scales as an unwelcome imposition.

The present invention provides a significant improvement in providing clinicians with the option to complete various different rating scales at appropriate times within a consultation. If a user selects a rating scale, the personalised medicine system 1, 100 automatically populates graphical representations of different symptoms/side effects, the slider for each symptom/side effect being based only on the relevant parts of the completed rating scale. Accordingly, the clinician can immediately see in an intuitive way how a patient scores in different symptoms/side effects (or categories thereof), assisting the clinician to gain a clearer understanding of the patient's condition.

Moreover, it is unnecessary for the clinician to complete a full scale to populate the sliders, allowing clinicians to cut down the lengthy process of completing full scale assessments by assessing only relevant items.

If the clinician completes both the PANSS and the Calgary scale, for example, part of the PANSS assessment will be used to populate some sliders (such as for anxiety) and part of the Calgary scale assessment will be used to populate other sliders (such as for depression and suicidality). Although both scales could be used to populate the depression slider, for example, only the relevant section of the Calgary scale is used in practice.

Because the personalised medicine system 1, 100 ranks the different scales in order of their importance to the various characteristics (symptoms/side effects) and uses only the relevant part of each relevant scale to populate the respective sliders, the clinician can be confident that he is viewing the fullest picture for each category. Thus, the present invention has a synergistic effect in using only parts of predetermined scales and ranking the respective scales for each characteristic, to provide the clinician with detailed information in a useful manner.

Moreover, by providing three or more indicators on each slider, the present invention allows clinicians to see changes in the various characteristics as treatment progresses. The functionality of allowing the user to update or input values for each characteristic is particularly useful. If the user does not have time within a consultation to carry out a full (or even partial) rating scale assessment, he can quickly view the position at the last consultation and, based on impressions formed and knowledge gained in the current consultation, he can very quickly update the positions on the sliders to reflect the patient's current state. Alternatively, even if the user has carried out a full or partial rating scale assessment, he can update the slider in view of additional information in his possession which he considers is not fully taken account of in the scale.

Similarly, the functionality of allowing the patient to update or input values for each characteristic is very useful. It allows patients to rate changes in characteristics in a meaningful way and provides useful feedback to the clinician in addition to the personalised medical system-populated values and the clinician-input values. It also provides the patient with useful feedback and may encourage them in feeling they have a measure of control over their illness and the treatment of it.

Thus, the personalised medicine system 1, 100 of the present invention offers a large improvement by providing the clinician with useful, easily updated and easily understood information that has not previously been available. As such, the personalised medicine system 1, 100 of the present invention drives up the quality of care to patients, and can help healthcare providers to lower their costs.

The foregoing description has been given by way of example only and it will be appreciated by a person skilled in the art that modifications can be made without departing from the scope of the present invention.

For example, the foregoing description describes operation of the personalised medicine system 1, 100 by reference to application to the treatment of mental illness. However, the present invention is not limited to this and can be applied across the spectrum of medical complaints. Although applicable to general practice and any illness or condition, the present invention has particular benefit in the treatment of longer term illnesses and conditions, which require on-going treatment and many consultations.

Although the present invention has been described by reference to consultations and visits by the patient to the clinician, it should be understood that consultations need not be face to face (although this is preferred). Moreover, as discussed above, the personalised medicine system 1, 100 may make provision for "dummy" visits/consultations, for example involving contact of the patient with other medical professionals or for adding the results of lab tests and other investigations.

Where the words "step" and "stage" have been used in this specification, they should not be construed narrowly and, except where the context requires otherwise, they may be considered to be synonymous. Thus, the terms incorporate nodes and sub-nodes as described above. Thus, each page could be considered as being associated with a separate, individual stage or step in the workflow, with these stages or steps being grouped into nodes and higher level stages or steps.

**Claims**

1. A personalised medicine system comprising:

   a processor;
   memory;
   an input module; and
   an output module,
   said personalised medicine system being configured:
   to provide a plurality of predetermined patient assessment routines;
   to display a plurality of pages associated with respective stages in a workflow for guiding a clinical consultation;
   in response to an input from a user on a displayed page, to receive data for a selected patient assessment routine;
   to determine a value for at least one patient characteristic based on the received data; and
   to display a respective graphical scale for each of a plurality of patient characteristics,
   wherein an indicator is displayed on each graphical scale at a position corresponding to at least one of the determined characteristic value and a characteristic value directly input by the user.

2. A personal medicine system according to claim 1, configured to display on each graphical scale both a first indicator at a position corresponding on the determined characteristic value, if available, and a second indicator at a position corresponding to the directly input characteristic value, if available.

3. A personal medicine system according to claim 1 or claim 2, further configured:

   to store at least one previously set characteristic value for each graphical scale; and
   additionally to display a third indicator for the previously set patient characteristic value on each graphical scale.

4. A personalised medicine system according to any one of the preceding claims, wherein at least one said patient assessment routine comprises a predetermined rating scale, the received data for the patient assessment routine comprises a number between predetermined limits in response to each question in the rating scale that is answered, and the determined value for a characteristic corresponds to the sum of the numbers input in response to questions relevant to the respective characteristic.

5. A personalised medicine system according to claim 4, wherein the patient assessment routine includes questions relevant to more than one characteristic.

6. A personalised medicine system according to claim 4 or claim 5, wherein more than one patient assessment routine includes a question that is relevant to a respective characteristic.

7. A personalised medicine system according to claim 6, wherein:

   for each respective characteristic for which there is more than one patient assessment routine that includes a relevant question, the patient assessment routines are ranked in order of importance, and
   if at least two patient assessment routines that include relevant questions have been completed, the data input in respect of the assessment routine ranked as most important for the characteristic are used to calculate the determined characteristic value.

8. A personalised medicine system according to claim 7, wherein the patient assessment routines are ranked in order of the largest possible sum of numbers that can be input in response to questions relevant to the respective characteristic.

9. A personalised medicine system according to any one of the preceding claims, wherein the indicator is displayed at a position corresponding to a normalised value of the determined characteristic value expressed as one of a ratio and a percentage of the maximum characteristic value that can be determined during the patient assessment routine.

10. A personalised medicine system according to any one of the preceding claims, configured such that the user drags the indicator along the graphical scale to directly input the characteristic value.

11. A personalised medicine system according to claim 10, configured to display the indicator at a position based on the determined characteristic value before the user directly inputs the characteristic value.

**12.** A personalised medicine system according to any one of the preceding claims, further configured, in response to a user input, to display when an assessment routine used to position the indicator is uncompleted.

**13.** A personalised medicine system according to any one of the preceding claims, wherein:

at least one patient assessment routine comprises input of physical examination data, and
the value determined for the at least one patient characteristic is based on a difference between previously input physical examination data and currently input physical examination data.

**14.** A method comprising:

providing a plurality of predetermined patient assessment routines;
displaying a plurality of pages associated with respective stages in a workflow for guiding a clinical consultation;
in response to an input from a user on a displayed page, receiving data for a selected patient assessment routine;
determining a value for at least one patient characteristic based on the received data; and
displaying a respective graphical scale for each of a plurality of patient characteristics, wherein an indicator is displayed on each graphical scale at a position corresponding to at least one of the determined characteristic value and a characteristic value directly input by the user.

**15.** A computer program stored on a computer-readable medium for causing a computer to carry out a method comprising:

providing a plurality of predetermined patient assessment routines;
displaying a plurality of pages associated with respective stages in a workflow for guiding a clinical consultation;
in response to an input from a user on a displayed page, receiving data for a selected patient assessment routine;
determining a value for at least one patient characteristic based on the received data; and
displaying a respective graphical scale for each of a plurality of patient characteristics, wherein an indicator is displayed on each graphical scale at a position corresponding to at least one of the determined characteristic value and a characteristic value directly input by the user.

## Fig. 1

Fig. 2

## Fig. 3

| Workflow step | Workflow node | Page ID | Activity | Base |
|---|---|---|---|---|
| 1. Review Data | Electronic data exchange | P004 | Electronic data exchange | |
| | Review | P001 | Reviewing actions from last time | |
| | | P002 | View actions due this time | [KB01] |
| | | P003 | Reviewing any documentation from external sources | |
| 2. Assess Patient Status | Conduct clinical interview | P005 | Updating patient profile | [KB20] |
| | Review smartphone data | P007 | Reviewing smartphone data | |
| | Assess efficacy/examine mental state | P008 | Completing symptom rating scales | [KB03] |
| | | P009 | Recording symptom severity | |
| | Assess tolerability/physical state/side effects | P010 | Completing side effect rating scales | [KB05] |
| | | P011 | Doing a physical exam | |
| | | P012 | Recording side effect severity | |
| | Assess adherence | P013 | Reviewing Smartphone adherence data | |
| | | P013 | Evaluating and recording adherence | [KB06] |
| | Assess patient preferences | P014 | Understanding patient and carer options | [KB19] |
| 3. Formulate Management Plan | Review working diagnosis | P015 | Selecting a primary diagnosis and co-morbidities | [KB21] |
| | Determine pharmacological treatment | P016 | Deciding whether or not to change medication regimen and recording reasons for changes | [KB07] |
| | | P017 | Doctor determines how he wants to make changes to AP medication | |
| | | P018 | Using the treatment selection wizard to guide AP selection – view side effect relative risk | [KB08] |
| | | P019 | Viewing drug guidance matrix | [KB11] |
| | | P020 | Considering dug options | [KB12] |
| | | P028 | Selecting drugs | [KB22] |
| | | P021 | Confirming and prescribing drugs | |
| | Determine investigations | P022 | Determining investigations | [KB14] |
| | Order investigations | P022 | Ordering investigations | |
| | Determine non-pharmacological treatment | P023 | Selecting psychological interventions | [KB15] |
| | | P023 | Selecting social / lifestyle interventions | |
| | | P023 | Selecting medical interventions | |
| 4. Additional Patient Centred Interventions | Smartphone | P024 | Providing patient with smartphone app or updating it | |
| | Patient engagement and education | P025 | Providing patient with additional information / sources of support | [KB16] |
| | Homework | P025 | Providing patient with homework (3rd-party resources) | |
| 5. Wrap up | Next appointment | P026 | Set date of next appointment | [KB17] |
| | | P026 | Review reminders for the next appointment, add any others | |
| | Communication with healthcare professionals | P027 | Sharing information | [KB18] |
| | Data exchanges to EHR | P027 | Updating Electronic Health Record (if relevant) | |
| | Electronic data exchange | | Electronic data exchange | |

## Fig. 4

60

65

John SMITH ID#12345 DOB 10/15/1982 (30) Male | Visit 08/20/2012

〉 ∨ Review Data 〉 〉 Assess Patient Status 〉 〉 Formulate Management Plan 〉 〉 Wrap-Up 〉 Complete Visit

### Review Data

| Electronic Data Exchange | Review | Interim Contacts |
|---|---|---|
| | Actions From Last Visit | External Documents |
| | Actions Due | Record Changes |
| | Open Monitoring Schedule | |

**Interim Contacts**

∨ External Documents

Has anything changed since John Smith's last visit?
⦿ unchanged  ○ changed

**Guided Recommendations**

∨ Knowledge Base Recommendations

**NICE guidelines**

Labs     Lipid Profile (last result on 06/05/2012)

P003

## Fig. 5

| ∨ Actions From Last Visit | Date of Last Visit **07/20/2012** |
|---|---|
| **Antipyschotic Medication** | Risperidone 4mg od – Increased due to lack of efficacy |
| **Other Medication** | Fluoxetine 20mg od – commenced |
| **Labs** | Lipid<br>Glucose |
| **Physical** | BP **120/80**<br>Weight **57kg** |
| **Rating Scales** | PANSS<br>Total **79**<br>Positive symptoms **18**<br>Negative symptoms **13**<br>General Psychopathology **48** |
| **Remote Monitoring** | Adherence monitoring |
| **Non-pharmacological** | Not included this time as no activities last time |

P001

## Fig. 6

| Actions Due | Date Actions Due **08/20/2012** |
|---|---|
| **Labs** | Liver function test |
| **Physical** | Weight |
| **Rating Scales** | BPRS |
| **Non-pharmacological** | Set up art therapy class |

P002

## Fig. 7

| Actions From Last Visit | Date of Last Visit **07/20/2012** |
|---|---|
| **Antipyschotic Medication** | Risperidone 4mg od - Increased due to lack of efficacy |
| **Other Medication** | Fluoxetine 20mg od - commenced |
| **Labs** | Lipid<br>Glucose |
| **Physical** | BP 120/80<br>Weight 57kg |
| **Rating Scales** | PANSS<br>Total 79<br>Positive symptoms 18<br>Negative symptoms 13<br>General Psychopathology 48 |
| **Remote Monitoring** | Adherence monitoring |
| **Non-pharmacological** | Not included this time as no activities last time |

| Actions Due | Date Actions Due **08/20/2012** |
|---|---|
| **Labs** | Liver function test |
| **Physical** | Weight |
| **Rating Scales** | BPRS |
| **Non-pharmacological** | Set up art therapy class |

**Guided Recommendation**

| Knowledge Base Recomm |  |
|---|---|
| **NICE guidelines** | |
| **Labs** | Lipid Profile (L |
| **Physical** | BP<br>Weight |
| **Rating Scales** | PANSS |

| mehealth™ Schizophreni |
|---|

Labs Glucose (Last result on 06/(

Consider repeating now on the
SMITH's profile

**Family History** Diabetes
**Last Glucose** 105 mg/dl

**Open Monitoring**

P001

P002

50

**Conduct Clinical Interview**

☐ External Documents

In order for mehealth to take into account John Smith's personal profile and past treatments, please take time on first visit to work through the sections provided below and complete details of John Smith's profile.

## ∨ Record Changes

| | |
|---|---|
| Diagnosis | Record Diagnosis |
| Antipsychotic Treatments | Record Antipsychotic Medications |
| Pyschotropic Treatments | Record Pyschotropic Medications |
| Contact | Record Contact(s) |
| Rating Scales | Record Rating Scale(s) |
| Physical Exams | Record Physical Exam(s) |
| Lab Results | Record Lab Results |
| Non-Pharmacological Treatments | Record Non-Pharmacological Treatments |

**Document Quick Add**

Quickly add any supporting documentation about John Smith, here.

**File name:**

Continue to John Smith's Profile >>

---

**Guided Recommendations**

∨ Knowledge Base Recommendations

**NICE guidelines**

| | |
|---|---|
| Labs | Lipid Profile (Last result on 06/05/2012) |
| Physical | BP |
| | Weight |

**Rating Scales** PANSS

∨ mehealth™ Schizophrenia Recommendations

**Labs** Glucose (Last result on 06/05/2012)

Consider repeating now on the basis of John SMITH's profile

**Family History** Diabetes
**Last Glucose**   105 mg/dl

**Open Monitoring Schedule**

50

Fig. 8

EP 2 782 056 A1

P003

29

# Fig. 9

**60** **65** **50**

## Fig. 10

**50**

**60**

**65**

| 👤 | ⟩ Review Data | ∨ Assess Patient Status | ⟩ Formulate Management Plan | ⟩ Wrap |

Conduct Clinical Interview  >  **Examine Mental State**  >  Assess Physical Status  >  Assess Adherence  >  Assess Patient Preferences

### Examine Mental State

| ∨  Symptom Rating Scales |

To record symptoms you may use the rating scales below and/or select the
Symptom Severity Mixer.

**∨ Complete Rating Scales**

Positive & Negative Symptoms

| PANSS | Defer | View Past Results |
| BPRS | Defer | View Past Results |

General Level of Functioning

| GAF | Defer | View Past Results |
| CGI | Defer | View Past Results |

Affective Symptoms

| CDSS | Defer | View Past Results |
| YMRS | Defer | View Past Results |

Side Effect Rating Scales > >

**∨ Past Rating Scale Results**

**Guided Recor**

| ∨  Knowledg |

**NICE guideline**

Labs

**Physical**

**Rating Scales**

| ∨  mehealth |

**Labs Glucose (L**

Consider repeati
SMITH's profile

**Family History
Last Glucose**

**Ope**

| ∧  Symptom Severity Mixer |

**P008**

# Fig. 11

Positive & Negative Syndrome Scale
(PANSS)

Instructional text here … …

| | Absent | Minimal | Mild | Moderate Severe | Moderate | Severe | Extreme |
|---|---|---|---|---|---|---|---|
| Positive Scale (P) | 0 | 1 | 2 | 3 | 4 | 5 | 6 |
| **Delusions**<br>Beliefs which are unfounded, unrealistic, and idiosyncratic.<br>Basis for rating:<br>Thought content expressed in the interview and its influence on social relations and behavior. | ● | ● | ● | ● | ● | ● | ● |
| **Conceptual disorganization**<br>Disorganized process of thinking characterized by disruption of goal-directed sequencing, e.g., circumstantiality, tangentiality, loose associations, non sequiturs, gross illogicality, or thought block. Basis for rating: Cognitive-verbal processes observed during the course of interview. | ● | ● | ● | ● | ● | ● | ● |
| **Excitement**<br>Beliefs which are unfounded, unrealistic, and idiosyncratic.<br>Basis for rating:<br>Thought content expressed in the interview and its influence on social relations and behavior. | ● | ● | ● | ● | ● | ● | ● |

| | Absent | Minimal | Mild | Moderate Severe | Moderate | Severe | Extreme |
|---|---|---|---|---|---|---|---|
| Negative Scale (N) | 0 | 1 | 2 | 3 | 4 | 5 | 6 |
| **Blunted affect**<br>Diminished emotional responsiveness as characterized by a reduction in facial expression, modulation of feelings, and communicative gestures. Basis for rating: observation of physical manifestations of affective tone and emotional responsiveness during the course of interview | ● | ● | ● | ● | ● | ● | ● |

# Fig. 12

75       72    74                                     70

Symptom Severity Mixer

**Positive Symptoms**

☐ adjust

**Negative Symptoms**

☐ adjust

**Cognition**

☐ adjust

**Suicidality**

☐ adjust

**Anxiety**

☑ adjust

**Depression**

☐ adjust

**Mania**

☐ adjust

76

P009

# Fig. 13

⌄ Side Effect Rating Scales

To record symptoms you may use the rating scales below and/or select the Side Effect Severity Mixer.

⌄ Complete Rating Scales

**AIMS**    *Defer*

**ACS**    *Defer*

**LUNSERS**    *Defer*

**Previous Results**

P010

# Fig. 14

**50**

**65** **60**

John SMITH ID#12345 DOB 10/15/1982 (30) Male | Visit 08/20/2012

> Review Data | ~ Assess Patient Status | > Formulate Management Plan | > Wrap-Up | Complete Visit

Conduct Clinical Interview > Patient Profile > Examine Mental State > **Assess Physical Status** > Assess Adherence > Assess Patient Preferences

## Assess Physical Status

∨ Physical Exam

Instructional text here ...

**Guided Recommendations**

**Vital Signs**

| | | |
|---|---|---|
| Pulse | _bpm_ | Defer |
| Blood Pressure | _mm/Hg or cm/Hg_ | Defer |
| Temperature | _f or c_ | Defer |

∨ Knowledge Base Recommendations

**Body Weight & Height**

| | | |
|---|---|---|
| Body weight | _kg or lbs_ | Defer |
| Height | _feet/inches or m_ | Defer |
| BMI | _kg/m2_ | Defer |

∨ mehealth™ Schizophrenia Recommendations

**Other Examinations**

| | | |
|---|---|---|
| Eye examination | normal ∨ | Defer |
| Dental examinations | abnormal ∨ | Defer |

**Screenings**

| | | |
|---|---|---|
| Symptoms of hyperprolactinemia | select ∨ | Defer |
| Abnormal involuntary movements | abnormal ∨ | Defer |
| Extrapyramidal side effects | normal ∨ | Defer |
| Extrapyramidal side effects, including akathisia | abnormal ∨ | Defer |

**Labs**

| | | |
|---|---|---|
| ECG/EKG | select ∨ | Defer |
| EEG | abnormal ∨ | Defer |
| Imaging/EEG - CT | normal ∨ | Defer |
| Imaging/EEG - MRI | abnormal ∨ | Defer |

Save Physical Exam

∨ Physical Exam Results

P011

## Fig. 15

80

Side Effect Severity Sliders

**Weight Gain**

**Glucose Intolerance/Diabetes**

**Hyperlipidemia**

**Breast Enlargement/Galactorrhea**

**Amenorrhea/Dysmenorrhea**

**Other Sexual Side Effects**

**Seizures**

**Dystonia**

P012

# Fig. 16

60          65

| John SMITH  ID#12345   DOB 10/15/1982 (30)  Male | Visit 08/20/2012 | | | | |

〉 Review Data 〉 〉 Assess Patient Status 〉 ✓ Formulate Management Plan 〉 〉 Wrap-Up 〉   Complete Visit

**Working Diagnosis** > Pharmacological Treatment > Labs > Non Pharmacological Treatment > Smartphone Data > Patient Engagement & Education

∨ Select Primary Diagnosis

Please select the current diagnosis by clicking the relevant button.

| First episode psychosis | Schizophrenia |
| Affective psychosis | Non-affective psychosis |

| Manic | Mixed affective | Bipolar | Not bipolar |

Save Primary Diagnosis 〉

∨ Select Co-morbidities

Please select any of the co-morbidities from the list below.

☐ Anxiety
☐ Sleep disturbance
☐ Substance abuse
☐ Alcohol abuse

Save Co-morbidities 〉

**Guided Recommendations**

∨ Knowledge Base Recommendations

∨ medhealth™ Schizophrenia Recommendations

P015                                                    50

# Fig. 17

**60**  **65**

John SMITH  ID#12345  DOB 10/15/1982 (30)  Male                    Visit 08/20/2012

> Review Data    > Assess Patient Status    ⌄ Formulate Management Plan    > Wrap-Up      Complete Visit

Working Diagnosis > Pharmacological Treatment > Labs > Non-Pharmacological Treatment > Smartphone Data > Patient Engagement & Education

⌄ Current Drug Treatment

Taking into account the efficacy, tolerability, adherence and patient preferences, please use the Treatment Configurator below to make any required changes or select "No Change" to continue with the John Smith's current treatment regimen.

Risperidone, 4mg od
Chlorpromazine, 200mg od
Antidepressant, 50mg od

No Change

⌄ Change Drug Treatment Selection

Please add, remove, review and complete drug treatment selection below.

**Selected Antipsychotics:**

Risperidone    4 mg ▼      od ▼      oral ▼    discontinue
+ Add Antipsychotic

**Selected Psychotropic Drugs:**

Chlorpromazine    200 mg ▼     od ▼      oral ▼    discontinue
+ Add Psychotropic

**Selected Other Drugs:**

Antidepressant    50 mg ▼     od ▼      oral ▼    discontinue
+ Add Other

Save Treatment Selection

⌃ Treatment Selection Wizard

Guided Recommendations

⌄ Knowledge Base Recommendations

⌄ mehealth™ Schizophrenia Recommendations

**68**                    P016                    **50**

# Fig. 18

^ SIDE EFFECT RELATIVE RISK

Saeed Choudry's side effect risk profile has been analyzed based on the information you provided.

- Hover or click on each side effect for details.
- Risk factors have been identified from an expert panel review of the evidence base. Click the ℹ️ to explore the relevant evidence base.
- Side effects are ranked according to this patient's risk relative to other patients.
- Characteristics which increase this patient's risk are detailed.

ℹ️ **Dystonia**

ℹ️ **Agranulocytosis**

ℹ️ **Hyperlipidemia**

ℹ️ **Weight Gain**

ℹ️ **Parkinsonism**

ℹ️ **Sedation**

ℹ️ **Akathisia**

ℹ️ Glucose Intolerance/Diabetes 🚫

Please select the side effects you would like to avoid.
For example, akathisia may be particularly undesirable in a patient who is agitated.

P018

39

# Fig. 19

∨ Drug Selection Wizard

Taking into account **John Smith**'s side effect risk and symptoms the
drug selection wizard has matched these 10 most commonly prescribed
oral and injectable antipsychotics.

Discuss Selected Drugs >

### ∨ Top 10 Antipsychotics

Customized Oral List

| Drug Name | NICE | United Healthcare | Maudsley | Mass Health | Drug Info | Cost |
|-----------|------|-------------------|----------|-------------|-----------|------|
| ☐ quetiapine | ● | ● | ● | | 𝒊 | $$ |
| ■ aripiprazole | ○ | ○ | ○ | | 𝒊 | $$ |
| ☐ risperidone | ● | ● | ● | ● | 𝒊 | $$ |
| ■ olanzapine | ○ | ○ | ○ | | 𝒊 | $ |
| ☐ clozapine | | | | | 𝒊 | $$ |
| ■ asenapine | ○ | ○ | ○ | | 𝒊 | $ |
| ☐ ziprasidone | ● | ● | ● | | 𝒊 | $ |
| ■ fluphenazine | ○ | | | | 𝒊 | $$ |
| ☐ lurasidone | ● | ● | ● | | 𝒊 | $$ |
| ■ perphenazine | ○ | | | | 𝒊 | $$$ |

Customized Injectable List

| Drug Name | NICE | United Healthcare | Maudsley | Mass Health | Drug Info | Cost |
|-----------|------|-------------------|----------|-------------|-----------|------|
| ☐ haloperidol | | | | | 𝒊 | $$ |
| ■ flupentixol | | | | | 𝒊 | $$ |
| ☐ risperidone | | | | | 𝒊 | $$ |
| ■ zuclopenthixol | | | | | 𝒊 | $$ |

P019

## Fig. 20A

EP 2 782 056 A1

## Fig. 20B

Select Visit Type

Select Visit Date

2/26/2013

Start >

42

## Fig. 20C

| Visit | Anitpsychotic Medication | Other Medications | Physical | Labs | Rating Scales | Mobil Applicati |
|---|---|---|---|---|---|---|
| Start | →| | →| | →| | →| | →| | |
| 10-Jul-12 | Risperdone 4 mg od | Fluxetine 4 mg od | ▼ BP / ▲ Weight | ► Lipids / ▼ Glucose | | Adherer Monitor |
| 29-Jun-12 | | | ► Screening: Extrapyrimidal Side Effects | ▲ EKG | ▲ BPRS / ▼ GAF | |
| 18-May-12 | Olanzapine 10 mg od | | | ► EKG | ► PANSS | |
| 30-Dec-11 | Seroquel 4 mg od | | | ► EKG | ► CDSS | |

470

400

## Fig. 21

Alan BROWN ID#00014 DOB 02/08/1993 (19) Male

> Review Data  > Patient Status  > Formulate Management Plan  > Wrap-Up  Complete Visit

Data Exchange > Review

Actions from Last Visit          Date of Last Visit

Guided Recommendations

Actions Due

Documentation

> Expand

| Visit | Anitpsychotic Medication | Other Medications | Physical | Labs | Rating Scales | Mobile Applications |
|---|---|---|---|---|---|---|
| 28-Aug-12 | Drugname 4 mg od | Drugname dose ▼ | Pulse / BMI | | PANSS | |

447

445

450

# Fig. 22

200

| Clinical Team Input 210 | Electronic Health Record 212 | Patient Portal 214 | Investigation Results 216 |

Patient Data Store 230

Rules Engine 270

User Interface 280

Knowledge Base Store 260

| Guidelines 1-n 240 | Evidence Base Synthesis 242 | Peer Consensus 244 | Prescribing Data 246 | Licensed Content 248 | Clinical/ Research Datasets 250 |

**Fig. 23A**

300 Antipsychotic treatment algorithm

A

310 +History of atypical antipsychotic failure

322 –Only one atypical

B

312 Trial of atypical

324 Trial of different atypical

338 –Historical non-compliance

340 +Depot of halperidol or fluphenazine

344 -Depot of halperidol or fluphenazine

342 Trial of halperidol or fluphenazine

346 –No response to depot

348 +Different atypical following depot

352 -Different atypical following depot

350 Trial of different atypical

354 –No response to atypical following depot

356 +Trial of clozapine

360 -Trial of clozapine

364 –No response to clozapine

358 Trial of clozapine

362 –Partial response to clozapine

366 Combination

368 +No response to clozapine + augmenting agent

372 -No response to clozapine + augmenting agent

368 Clozapine + augmenting agent

374 Combination

## Fig. 23B

320 -History of atypical antipsychotic failure

330 +Only one atypical

A

B

332 -Only two atypicals

380 +Only two atypicals

386 -Typical trialled

382 +Typical trialled

384 Trial typical

334 +Historical non-compliance

336 Trial of different atypical

388 -No response to typical

390 +Trial of clozapine

393 -Trial of clozapine

392 Trial of clozapine

399 -No response to clozapine

394 -Partial response to clozapine

400 Combination

395 +No response to clozapine + augmenting agent

397 -No response to clozapine + augmenting agent

396 Clozapine + augmenting agent

398 Combination

# Fig. 24

| Month | Trigger which sets/resets clock | Visit | WF | Page | Para/ Page of guideline | How communicated in Guideline area | Secondary wording |
|---|---|---|---|---|---|---|---|
| NICE | | | | | | | |
| every 12 | 1st visit ever | n/a | 1,2<br>3<br>3 | 027<br>027<br>002 | 1.4.1.2 | Communications section : Send reminder to primary care team | GPs and other primary healthcare professionals should monitor the physical health of people with schizophrenia at least once a year |
| every 12 | 1st visit ever | n/a | 1,2,3 | 022 | 1.4.1.3 | Investigations section: People at risk of developing cardiovascular disease... | 1.4.1.3 |
| | | | | | | Physical Exam section: People at risk of developing cardiovascular disease... | 1.4.1.3 |
| 0 | 1st visit ever | n/a | 1,2 | 009 | 1.1.4.2 | Symptom Review section: Monitor for coexisting conditions, including depression and anxiety | Routinely monitor for other coexisting conditions, including... |
| 0-6 | | each visit | 3 | 009 | | | Routinely monitor for other coexisting conditions, including... |
| every 6 | | n/a | 3 | 009 | | | Routinely monitor for other coexisting conditions, including... |
| 0 and every 12 | 1st visit ever | n/a | 1,2,3 | 023 | 1.1.5.2 | Psychosocial Interventions section: Offer social, group and physical activities | All teams providing services for people with schizophrenia should... |
| 0 | 1st visit ever | n/a | 1,2 | 025 | 1.1.6.1 | Carer section: How to interact with carers | When working with carers of people with schizophrenia: • provide written and verbal information on schizophrenia and its management, including how families and carers can help through all phases of treatment • offer them a carer's assessment |

## Fig. 25

| | NICE | United | Maudsley | Risks associated with this patient's profile | mehealth recommen-dations | Your preferred frequency |
|---|---|---|---|---|---|---|
| **Investigations** | | | | | | |
| Lipid profile | 6 months | N/A | 6 months | - Family history of heart disease<br>- HDL > X | 3 months | |
| Glucose | 12 months | 12 months | N/A | | | |
| Liver function tests | ...etc. | | | | | |
| **Examinations** | | | | | | |
| Weight | | | | | | |
| BP | | | | | | |
| Dental exam | | | | | | |
| **Rating Scales** | | | | | | |
| BPRS | | | | | | |
| PANSS | | | | | | |

# Fig. 26

| Moderators of outcome/Risk factors | Variable categories | WG | AGRAN | AKATH | DIAB | TD | DYSLIPID |
|---|---|---|---|---|---|---|---|
| Age | <19 | y | | | | | y |
| | 20 to 30 | y | | | | | |
| | 31 to 45 | y | | | | | |
| | 45 to 64 | y | | | | | |
| | <21 | | y | | | | |
| | <40 | | | | | y | |
| | >40 | | y | | | y | |
| | >50 | | y | y | | | |
| | >60 | | y | | | y | |
| | <50 | | | y | | | |
| | <45 | | | | y | y | y |
| | > or = 45 | | | | y | | y |
| | ≥55 | | | | | | Y |
| | <55 | | | | | | Y |
| | 25 to 60 | | | | | y | |
| Gender | Male | y | y | y | y | y | y |
| | Female | y | y | y | y | y | y |
| Race | White alone | y | y | | y | y | |
| | non white/ non Caucasian | y | | | y | y | |
| | african – American | y | | | | | |
| | Hispanic | y | | | | | |
| | Indian | y | | | | | |
| | Pakistani | y | | | | | |
| | Chinese | y | | | | | |
| | Japanese | y | | | | | |
| | Other | y | | | | | |
| | Asian | | y | | | | |
| | African- Caribbean | | y | | | | |
| Acculturation (adoption of another culture) | Acculturation | | | y | | y | |
| | Maintenance of own culture | | | y | | y | |
| Marital status | Married | | | | | y | |
| | Single | | | | | y | |
| Employment status | Employed | | | | | y | |
| | not employed | | | | | y | |
| Smoking | Smoker | y | | | y | y | y |
| | Non-smoker | y | | | y | y | y |
| Alcohol | No alcohol | y | | | y | | |
| | Occasional | y | | | | | |
| | alcohol abuse | y | | | | y | |
| | Diagnosed alcohol dependence | y | | | | | |
| Alcohol intake | Heavy consumption or binge drinking | | | | y | | |
| | Light/moderate consumption (<30units a week) | | | | y | | |
| Acute alcohol intake | Acute intake >8 units | | | y | | | |
| | Lower acute intake | | | y | | | |
| Alcohol consumption | excessive (> 21 male/14 female units) | | | | | | Y |
| | units within recommended range | | | | | | Y |
| Psychoactive substances | Use of cocaine | | | y | | | |
| | Other | | | y | | | |
| Cannabis usage | Yes | y | | | | | |
| | No | y | | | | | |
| Drug usage | Yes | | | | | y | |
| | No | | | | | y | |

# Fig. 27

## WEIGHT GAIN INFLUENCES

| Moderator of outcome | Moderator Strength (Importance of moderator in overall prediction) | Category | Category Strength (Specific influence of variables within each moderator) | References |
|---|---|---|---|---|
| Age | 3 | <19 | 3 | 2 |
| | | 20 to 30 | 2 | 3 |
| | | 31 to 45 | 1 | 6 |
| | | 46 to 64 | 1 | 7 |
| | | >65 | 1 | 12 |
| | | | | 16 |
| Gender | 2 | male | 2 | 4 |
| | | female | 1 | 3 |
| | | | | 17 |
| | | | | 21 |
| Race | 3 | white alone | 2 | 3 |
| | | non white; | 3 | 22 |
| | | african - american | 2 | 26 |
| | | hispanic | 1 | 30 |
| | | indian | 1 | 43 |
| | | pakistani | 1 | 5 |
| | | chinese | no data | 44 |
| | | japanese | no data | |
| | | pima indians living in Arizona | 1 | 19 |
| | | pima indians living in Mexico | | |
| | | other | | |
| Smoking | 1 | Smoker | 2 | 14 |
| | | Non-smoker | 1 | |
| | | | | 19 |
| Alcohol | 1 | No alcohol | 1 | 26 |
| | | Occasional | 1 | |
| | | Alcohol abuse | 2 | |
| | | Diagnosed alcohol dependence | no data | |
| Cannabis usage | 2 | Yes | 2 | 22 |
| | | No | 1 | |
| INCREASED appetite? * This moderator occurs after Tx with AP * | 2 | Increased appetite | 3 | 3 |
| | | No increaased appetite | 1 | 11 |
| | | | | 19 |
| | | | | 24 |
| poor diet | 1 | Poor diet | 2 | 6 |
| | | Good diet | 1 | 16 |
| | | | | 22 |
| lack of exercise | 2 | Lack of exercise | 2 | 6 |
| | | Regular exercise | 1 | 16 |
| | | | | 22 |
| | | | | 31 |
| low levels of self control | 1 | Low self control | 2 | 11 |
| | | Higher self control | 1 | 22 |

# Fig. 28

## DRUG SIDE EFFECTS WEIGHTING TABLE

| Side Effect | Olanzapine | Risperidone | Amisulpride | Quetiapine | Aripiprazole | Clozapine |
|---|---|---|---|---|---|---|
| Hyperlipidemia | 2 | 3 | 1 | 5 | 4 | 2 |
| Breast Enlargement/ Galactorrhoea | 3 | 2 | 2 | 2 | 2 | 3 |
| Weight Gain | 5 | 3 | 1 | 3 | 2 | 5 |
| Glucose Intolerance/Diabetes | 5 | 3 | 2 | 3 | 1 | 5 |
| Amenorrhoea/ Dysmenorrhoea | 5 | 3 | 2 | 4 | 5 | 1 |
| Other Sexual Side Effects | 2 | 3 | 5 | 2 | 3 | 2 |
| Sedation | 3 | 1 | 4 | 1 | 3 | 3 |
| Hypotension | 1 | 5 | 3 | 5 | 1 | 1 |
| Seizures | 3 | 3 | 1 | 4 | 4 | 3 |
| Dystonia | 2 | 4 | 2 | 3 | 3 | 5 |
| Anticholingeric Side Effects | 5 | 4 | 3 | 2 | 1 | 4 |

## Fig. 29

| Slider | Source ranking number | Source name and relevant items | Scale range | Symptom score calculation method |
|---|---|---|---|---|
| Positive symptoms | 1 | PANSS positive symptoms subscale (i.e. the 7 items from the 'P' subscale) | Min = number of items completed Max = (7 * number of items completed) | Sum of the completed items. |
| | 2 | Use the following four items from BPRS: #4, Conceptual Disorganisation; #12, Hallucinatory Behaviour; #15, Unusual Thought Content; #11, Suspiciousness | Min = number of items completed Max = (7 * number of items completed) | Sum of the completed items. |
| Negative symptoms | 1 | PANSS negative symptoms subscale (i.e. the 7 items from the 'N' subscale) | Min = number of items completed Max = (7 * number of items completed) | Sum of the completed items. |
| | 2 | Use the following three items from the BPRS: #13, Motor Retardation; #16, Blunted Affect; #3, Emotional Withdrawal | Min = number of items completed Max = (7 * number of items completed) | Sum of the completed items. |
| General psychopathology symptoms | 1 | PANSS general psychopathology symptom subscale (i.e. the 16 items from the 'G' subscale) | Min = number of items completed Max = (7 * number of items completed) | Sum of the completed items. |
| Cognitive symptoms | 1 | Use the following five items from PANSS: P6, N5, N6, G2, and G10 | Min = number of items completed Max = (7 * number of items completed) | Sum of the completed items. |
| Anxiety | 1 | Use item G2 from PANSS | Min = 1 Max = 7 | Use the value (there's only item) |
| | 2 | Use item 2 from BPRS | Min = 1 Max = 7 | Use the value (there's only item) |
| Depression | 1 | Calgary (use all 9 items) | Min = 0 Max = 27 | Sum the scores. |
| | 2 | Use item G6 from PANSS | Min = 1 Max = 7 | Use the value (there's only item) |
| | 3 | Use item 9 from BPRS | Min = 1 Max = 7 | Use the value (there's only item) |
| Suicidality | 1 | Calgary, item 8 | Min = 0 Max = 3 | Use the value (there's only item) |
| Mania | 1 | Young Mania Rating Scale | 4 items have a min of 0 and a max of 8 7 have a min of 0 and a max of 4 | Sum the scores. |
| Sleep disturbance | 1 | [No prepositioning] | | [No calculation – doc can only ever drag the slider] |
| General level of functioning | 1 | GAF | 1-100 | Use the value (there's only item) |
| Global severity of illness | 1 | CGI, item 1 | Min = 1 Max = 7 | Use the value (there's only item) |

# Fig. 30

| Side effect | Lunsers | |
| --- | --- | --- |
| | Lunsers item(s) | Min-Max score |
| Weight gain | 39 | 0 -4 (ie 5 positions of slider = both ends and 3 in between) |
| Breast enlargement/galactorrhoea | 7 | 0-4 |
| Amenorrhoea/dysmenorrhoea | 50, 13 | 0-8 |
| Other sexual side effects | 17,24,46 | 0-12 |
| Dystonia | 34 | 0-4 |
| Parkinsonism | 29 | 0-4 |
| Akathisia | 40 | 0-4 |
| Sedation | 2,31,18 | 0-12 |
| Skin reactions | 1,35,47,49 | 0-16 |
| Constipation | 10 | 0-4 |

# Fig. 31

S1 Determine Context

↓

S2 Determine Evolving Treatment History

↓

S3 Determine Patient Characteristics

↓

S4 Display Advisory Information

## EUROPEAN SEARCH REPORT

Europäisches
Patentamt
European
Patent Office
Office européen
des brevets

**Application Number**

EP 13 15 9813

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/197660 A1 (PRODANOVICH SRDJAN [US]) 2 August 2012 (2012-08-02) * abstract; claims 1-21; figures 1-40 * * paragraphs [0002], [0023] - [0026], [0071] - [0248], [0326] - [0386], [0643], [0663] - [0681] * ----- | 1-15 | INV. G06Q10/06 G06Q50/22 G06F19/00 ADD. G06F3/0484 |
| X | US 2006/135859 A1 (ILIFF EDWIN C [US]) 22 June 2006 (2006-06-22) * abstract; claims 1-54; figures 1-26 * * paragraphs [0012] - [0058], [0095] - [0101], [0121] - [0132], [0159] - [0174], [0214] - [0220], [0225] * * paragraphs [0316] - [0322] * ----- | 1-15 | |
| X | US 2005/033608 A1 (SHERR JEREMY [GB]) 10 February 2005 (2005-02-10) * abstract; claims 1-19; figures 1-6 * * paragraphs [0002], [0043], [0145], [0154] - [0182], [0213] - [0242] * ----- | 1-15 | |
| A | US 2009/144089 A1 (HEYWOOD BENJAMIN [US] ET AL) 4 June 2009 (2009-06-04) * abstract; claims 1-19; figures 1-6 * * paragraphs [0006], [0009], [0015], [0023], [0041], [0056], [0059], [0060], [0092] - [0097] * ----- | 1-15 | TECHNICAL FIELDS SEARCHED (IPC) G06Q G06F |

The present search report has been drawn up for all claims

| Place of search | Date of completion of the search | Examiner |
|---|---|---|
| The Hague | 12 September 2013 | Streit, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 13 15 9813

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2013

| Patent document cited in search report | | Publication date | Patent family member(s) | | Publication date |
|---|---|---|---|---|---|
| US 2012197660 | A1 | 02-08-2012 | NONE | | |
| US 2006135859 | A1 | 22-06-2006 | EP | 1941444 A1 | 09-07-2008 |
| | | | US | 2006135859 A1 | 22-06-2006 |
| | | | WO | 2007050147 A1 | 03-05-2007 |
| US 2005033608 | A1 | 10-02-2005 | NONE | | |
| US 2009144089 | A1 | 04-06-2009 | AU | 2008310575 A1 | 16-04-2009 |
| | | | AU | 2008310576 A1 | 16-04-2009 |
| | | | AU | 2008310577 A1 | 16-04-2009 |
| | | | CA | 2702402 A1 | 16-04-2009 |
| | | | CA | 2702406 A1 | 16-04-2009 |
| | | | CA | 2702408 A1 | 16-04-2009 |
| | | | EP | 2210226 A1 | 28-07-2010 |
| | | | EP | 2211687 A1 | 04-08-2010 |
| | | | EP | 2211690 A1 | 04-08-2010 |
| | | | JP | 2011501276 A | 06-01-2011 |
| | | | JP | 2011501844 A | 13-01-2011 |
| | | | JP | 2011501845 A | 13-01-2011 |
| | | | US | 2009125333 A1 | 14-05-2009 |
| | | | US | 2009131758 A1 | 21-05-2009 |
| | | | US | 2009144089 A1 | 04-06-2009 |
| | | | US | 2013066652 A1 | 14-03-2013 |
| | | | WO | 2009049276 A1 | 16-04-2009 |
| | | | WO | 2009049277 A1 | 16-04-2009 |
| | | | WO | 2009049278 A1 | 16-04-2009 |

EPO FORM P0459

For more details about this annex : see Official Journal of the European Patent Office, No. 12/82

**EP 2 782 056 A1**

**Patent documents cited in the description**

- US 6868422 B